# EUROPEAN PATENT APPLICATION

(11) **EP 3 095 444 A1**
(43) Date of publication of application: **23.11.2016**
(21) Application number: 15168496.6
(22) Date of filing: 20.05.2015
(51) Int. Cl.: A61K 31/16, A61P 29/00

(54) **A METHOD OF TREATING PERIPHERAL INFLAMMATORY DISEASE**

(71) Applicant: Dublin City University, Dublin 9 (IE); UNIVERSITY COLLEGE DUBLIN, Dublin 4 (IE)
(72) Inventor: The designation of the inventor has not yet been filed
(74) Representative: Purdy, Hugh Barry

(57) **Abstract**

An active for use in the treatment or inhibition of an inflammatory disease associated with over-activation of Toll-like Receptor 4 (TLR4), Toll-like Receptor 2 (TLR2) and Myeloid differentiating protein 88 (Myd88) adaptor-like protein (Mal) while maintaining a subject's ability to respond normally to a pathogen, in which the active is an oleamide or a derivative thereof.

## Description

### Field of the Invention

The invention relates to treatment of peripheral inflammatory disease. In particular, the invention relates to methods for the topical treatment of peripheral inflammatory disorders, especially skin inflammatory disorders such as atopic dermatitis.

### Background to the Invention

Toll-like receptors (TLRs) are a family of pattern recognition receptors that recognize molecular patterns associated with microbial pathogens. TLRs play a fundamental role in the initiation of the immune response and are expressed on cells of the immune system, most notably, dendritic cells (DCs), macrophages and neutrophils. Ten human TLRs have been found to date and they individually have a distinct function in innate immune recognition as each TLR has the ability to recognise a specific ligand.

Signalling by TLRs via their TIR-domain involves five adaptor molecules and include: (1) myeloid differentiating protein 88 (MyD88), (2) MyD88 adaptor like/TIR domain-containing adaptor protein (Mal/TIRAP), (3) TIR-domain-containing adaptor inducing interferon-β/TIR-containing adaptor molecule-1 (Trif/TICAM-1), (4)Trif-related adaptor molecule/TIR-containing adaptor molecule-2 (TRAM/TICAM-2), and (5) sterile alpha motif (SAM), HEAT/Armidillo motif and TIR-containing adaptor protein (SARM). Some TLRs do not utilize the same set of adaptors and the adaptors chosen determine the transcriptional response induced following microbial recognition. This is a result of the activation of two major signalling cascades, namely the MyD88-dependent pathway and the MyD88-independent pathway.

MyD88-dependent pathway eventually leads to the activation of the transcription factor NF-κB, initiating the production of pro-inflammatory cytokines such as IL-6 and TNF-α. MyD88- independent pathway activates the transcription factor interferon regulatory factor-3, (IRF3). Ultimately IRF3 activation leads to the production of type-1 interferons, IFN-α and IFN-β, and other IFN inducible genes. The MyD88 independent pathway requires the recruitment of the adaptor Trif. Trif uses some shared and unique signalling molecules compared with MyD88. Trif allows for the activation of IRF3, NF-κB and also involved in the induction of apoptosis.

MyD88 plays an essential role in the signalling by all TLRs, with the exception of TLR3. Trif is the sole adaptor used by TLR-3 in the activation of IRF3 and NF-κB. Of significance to this invention disclosure is the fact that TLR2 and TLR4 are the only TLRs that require Mal/TIRAP in addition with MyD88 in order to activate the MyD88 dependant pathway. TLR4 is unique in that it utilises both MyD88 and Mal to activate NFκB and Trif and TRAM to activate IRF3, it is also the only known TLR that engages four TIR containing adaptors.

The relationships between the various adaptor molecules involved in TLR receptor signalling is a key focus for the development of effective therapeutics for inflammatory disease and TLR4 and TLR2 have been associated with such diseases as inflammatory bowel disease (IBD), rheumatoid arthritis (RA) and cardiovascular disease. Specifically, TLR2 and TLR4 are highly expressed in the synovial tissue of patients with RA and are associated with high levels IL-12 and up-regulation of TLR2 and TLR4 occurs in a mouse model of Crohn's disease and treatment with Vasoactive intestinal peptide (VIP) induced a decrease in these receptors ameliorating the disease. Furthermore, the compound rabeximod specifically suppresses TLR2 and TLR4 thereby suppressing arthritis severity in mice. It exerted this effect downstream of TLR2 and TLR4, however, its molecular target has not been identified. Another compound, TAK-242 (resatorvid), has been shown to selectively bind to TLR4 and subsequently disrupt the interaction of TLR4 with adaptor molecules Mal and TRAM thereby inhibiting TLR4 signal transduction helping to ameliorate inflammatory diseases including RA and IBD.

Approximately 165 million people worldwide have rheumatoid arthritis, approx. 5 million with inflammatory bowel disease, approx. 1-2 million with multiple sclerosis and approx. 100 million with type 2 diabetes. With between 28-50% of a patient population being non-responders, there is always a need for new anti-inflammatory drugs, particularly those with specific targets and minimal side effects on immune defences.

Oh YT et al. ("Oleamide suppresses lipopolysaccharide-induced expression of iNOS and COX-2 through inhibition of NF-κB activation in BV2 murine microglial cells", Neurosci. Letts. (2010), 474(3): 148-153) demonstrates the use of oleamide as a suppressor of pro-inflammatory mediators in lipopolysaccharide-stimulated neuronal microglia. The document describes that the oleamide blocks activation of p38, ERK, PI3-kinase/Akt, ROS accumulation and NF-κB activation and may provide beneficial effects in the treatment of inflammatory brain damage induced by microglial activation.

US Patent Application Publication No. US 2008/0096250 discloses that Myd88 knockout mice do not produce cytokines IL-6 and IL-12 when stimulated with lipopolysaccaride or MALP-2, which signal through TLR4 and TLR2, respectively. The method described involves the use of inhibitor polypeptides and inhibitor polynucleotides of Toll Interleukin-1 Receptor Adaptor Proteins (TIRAPs)/Mal which inhibit both the Myd88-dependent and Myd88-indpendent responses. The document further discloses that the TLR4 pathway plays a role in inflammatory diseases.

At present current therapies for inflammatory diseases are predominantly anti-cytokine therapies. A major drawback of these therapies is the decreased host immune defence against infection as many of the cytokines blocked by them are involved in the immune response to a variety of pathogens following activation of TLRs. For example, an increased risk of opportunistic infections has been associated particularly with neutralization of tumour necrosis factor-α.

It is an object of the present invention to overcome at least one of the above-referenced problems.

### Summary of the Invention

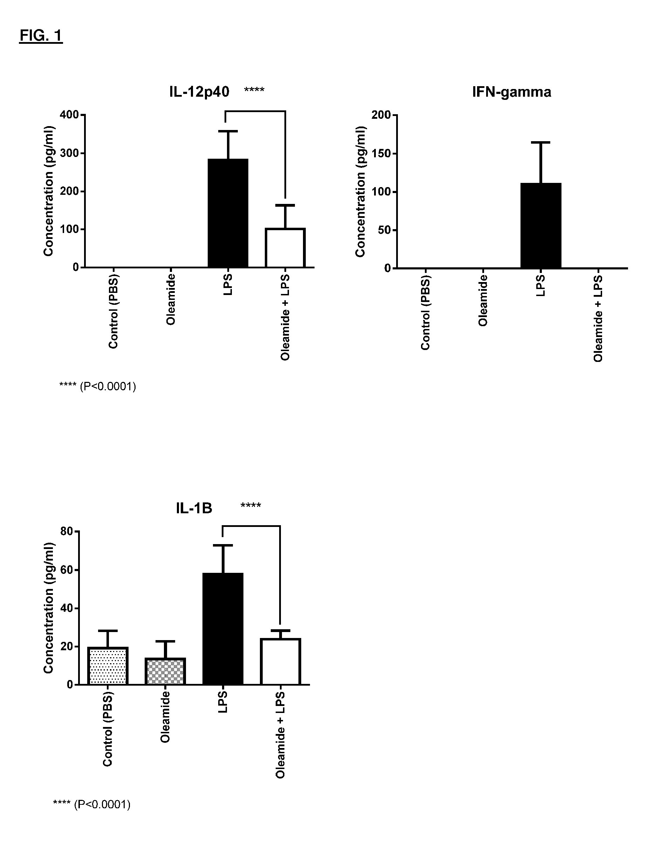

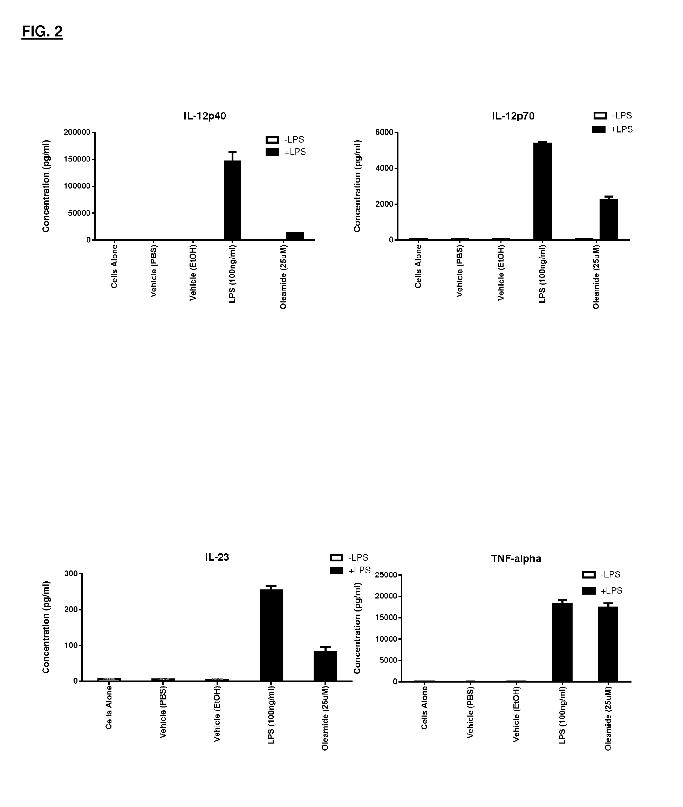

The Applicant provides *in-vivo* and *in-vitro* data demonstrating that certain fatty acids, amide derivatives of certain fatty acids, and analogues thereof, reduce the levels of certain pro-inflammatory cytokines, including IL-12p40, IL-23, IFN-gamma and IL-1β, in Bone Marrow Dendritic Cells (BMDCs) and BALB/c mice following LPS stimulation (Figs 1 and 2). Table 1 provides data showing a similar immunomodulatory effect for oleamide, palmitamide, arachidonamide, stearamide, palmitoleamide, linoleamide, and linolenamide, and immunomodulatory analogues thereof (Table 1).

In a first aspect, the invention provides a fatty acid based compound for use in a method of treating or preventing a peripheral inflammatory disorder in a mammal.

Preferably, the fatty acid based compound is a fatty acid selected from oleic acid, palmitoleic acid, stearic acid, arachidonic acid, linoleic acid and linolenic acid.

Preferably, the fatty acid based compound is an amide of a fatty acid based compound (hereafter "fatty acid amide").

Preferably, the fatty acid amide is selected from oleamide, palmitamide, arachidonamide, stearamide, palmitoleamide, linoleamide, and linolenamide.

Preferably, the fatty acid based compound is an immunomodulatory analogue of oleamide. Preferably, the immunomodulatory analogue of oleamide is selected from compounds 37, 38, 39, 40, 41, 43, 44, 69 and 104 of Table 1.

Preferably, the fatty acid based compound is an immunomodulatory analogue of palmitamide. Preferably, the immunomodulatory analogue of palmitamide is selected from compounds 78, 79, 80, 81, 82, 83, 84 and 85 of Table 1.

Preferably, the fatty acid based compound is an immunomodulatory analogue of arachidonamide. Preferably, the immunomodulatory analogue of arachidonamide is selected from compounds 65, 66, 67, 68, 92, 93, 94, 95 of Table 1.

Preferably, the fatty acid based compound is an immunomodulatory analogue of stearamide. Preferably, the immunomodulatory analogue of stearamide is selected from compounds 70, 71, 72, 73, 74, 75, 76 and 77 of Table 1.

Preferably, the fatty acid based compound is an immunomodulatory analogue of palmitoleamide. Preferably, the immunomodulatory analogue of palmitoleamide is selected from compounds 89, 90 91, and 101 of Table 1.

Preferably, the fatty acid based compound is an immunomodulatory analogue of linoleamide. Preferably, the immunomodulatory analogue of linoleamide is selected from compounds 53, 54, 58, 100, and 102 of Table 1.

Preferably, the fatty acid based compound is an immunomodulatory analogue of linolenamide. Preferably, the immunomodulatory analogue of linolenamide is selected from compounds 57, 59, 60, 62 99 and 103of Table 1.

Preferably, the peripheral inflammatory disease is an immune-mediated inflammatory disorder of the skin.

Suitably, the immune-mediated inflammatory disorder of the skin is selected from atopic dermatitis, contact dermatitis, acne and psoriasis.

In a preferred embodiment, the fatty acid based compound is administered topically to epithelial cells of a mammal, for example to the skin, lining of the airways, or lining of the gastrointestinal tract, of a mammal.

In another embodiment, the peripheral inflammatory disease is an immune-mediated inflammatory disorder of the joints. Preferably, the immune-mediated inflammatory disorder of the joints is rheumatoid arthritis.

In another embodiment, the peripheral inflammatory disease is an immune-mediated inflammatory disorder of the cardiovascular system. Typically, the immune-mediated inflammatory disorder is atherosclerosis.

In another embodiment, the peripheral inflammatory disease is an immune-mediated respiratory inflammatory disorder. Typically, the immune-mediated respiratory inflammatory disorder is selected from asthma and chronic obstructive pulmonary disease (COPD).

In another embodiment, the peripheral inflammatory disease is an immune-mediated inflammatory disorder of the intestinal tract. Typically, the immune-mediated inflammatory disorder of the intestinal tract is selected from inflammatory bowel disease (IBD)(colitis and crohn's disease).

The invention also provides a pharmaceutical composition comprising a fatty acid based compound and a suitable pharmaceutical excipient.

The invention also provides a pharmaceutical composition comprising a fatty acid based compound and a suitable pharmaceutical excipient, formulated for topical delivery to epithelial cells of a mammal, for example to the airways, or to the gastrointestinal tract, or to the skin of the mammal.

Typically, the pharmaceutical composition is formulated as a cream, ointment, paste, lotion, spray or gel.

The invention also relates to a pharmaceutical composition comprising oleamide or an immunomodulatory analogues thereof for use in a method of treating atopic dermatitis in a human.

The invention also relates to a patch or bandage comprising a pharmaceutical formulation of the invention and configured for attachment to the skin of a human and release of the pharmaceutical composition to the skin.

### Brief Description of the Drawings

Fig. 1: *In vivo* results: An *in vivo* study (LPS Shock Model) was performed on BALB/c female mice aged 17-19 weeks. The mice were divided into four groups: (1) mice administered PBS (control) via intraperitoneal (IP) injection, (2) mice administered 10 mg/kg of oleamide via IP injection, (3) mice administered 3µg lipopolysaccharide (LPS) via intravenous (IV) injection and (4) mice administered 10 mg/kg of oleamide via IP injection 2 hours before 3µg LPS IV injection. Six hours after LPS IV injection, each of the mice was culled and serum was collected to measure cytokine levels (IL-12p40, INF-gamma and IL-1β) by ELISA. (n=4)
Fig. 2: *In vitro* results: Bone marrow dendritic cells (BMDCs) were cultured *in vitro* and plated at 1x10⁶ cells per ml. The BMDCs were conditioned with 25uM oleamide 1 hour prior to stimulation with 100ng/ml LPS (*E.Coli* serotype R515). After 24 hours, the supernatants were removed and analysed for cytokine levels of IL-12p40, IL-12p70, IL-23 and TNF-alpha using specific immunoassays. (n=9)

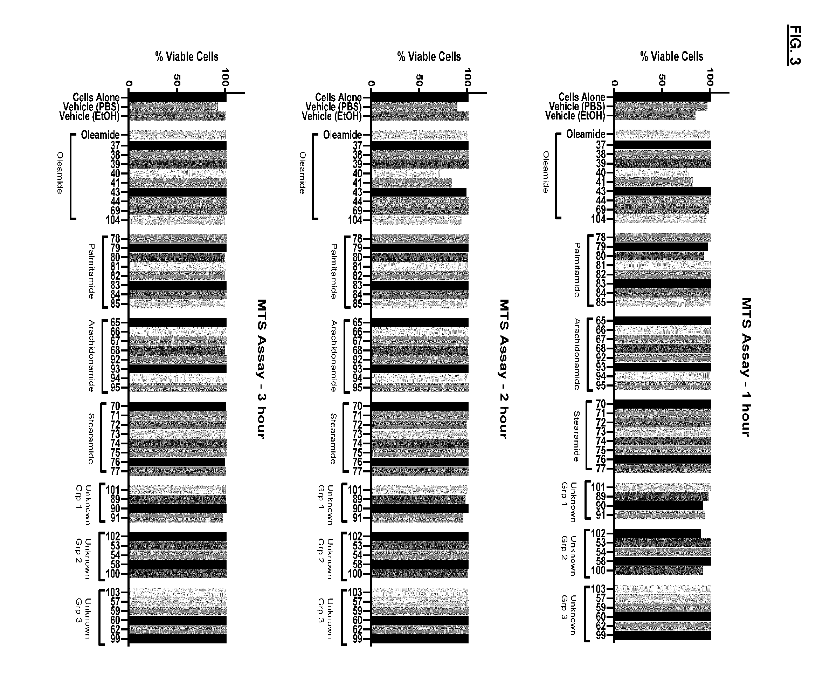

Fig. 3: *In vitro* cell viability results: Bone Marrow Dendritic cells (BMDCs) were cultured *in vitro* and plated at a concentration of 1 x 10⁶ cells per ml. An MTS assay was performed in order to determine the viability of the BMDCs when they were associated with oleamide and its family of analogues. (n=3)

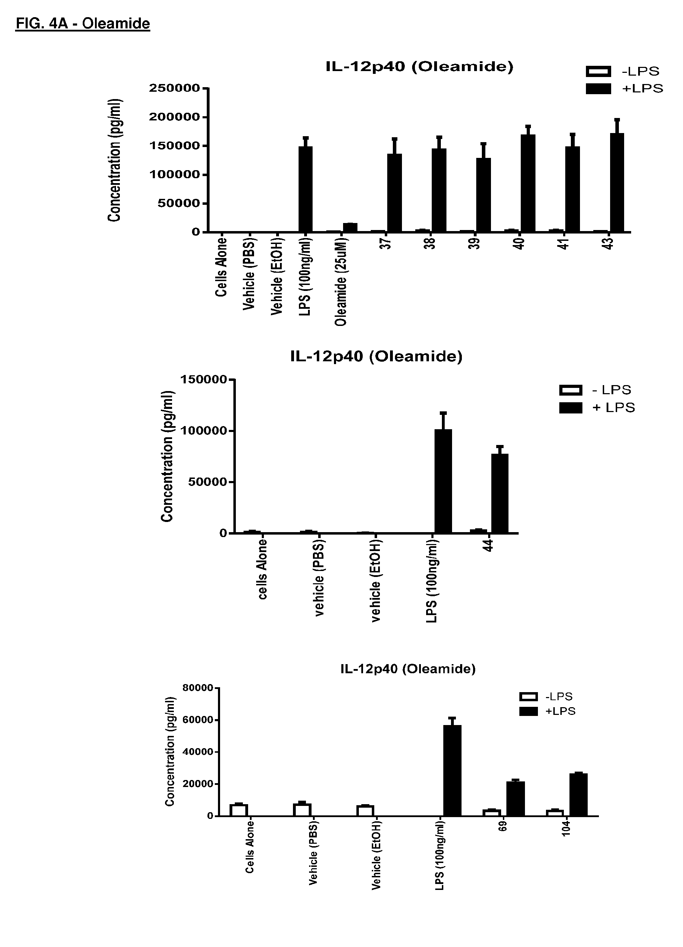

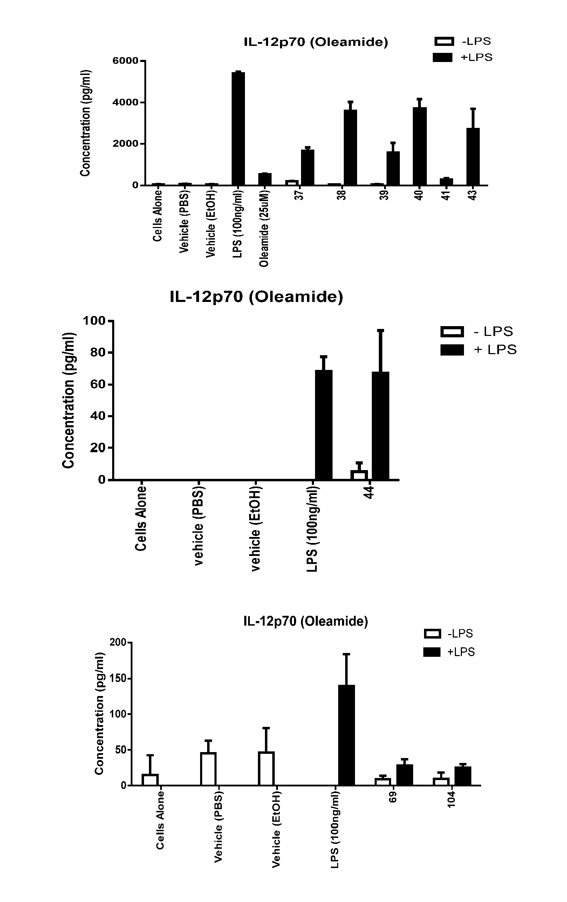

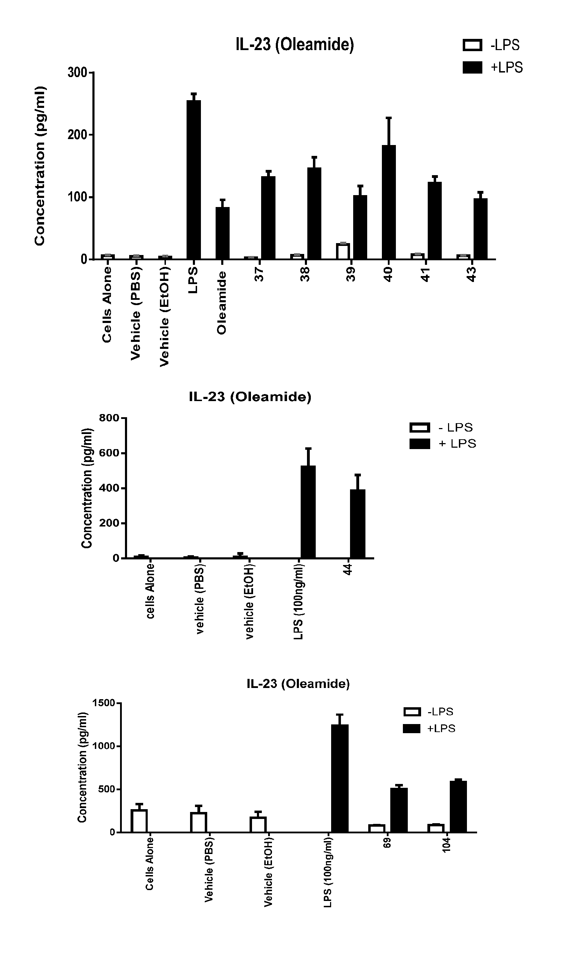

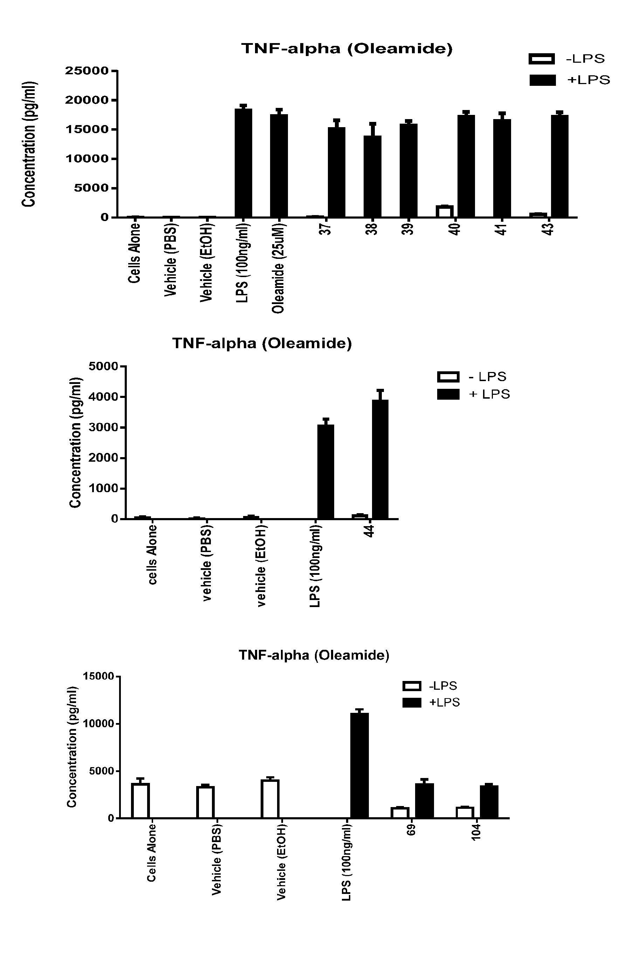

Figs 4(A) to 4(G): *In vitro* Results: Bone marrow dendritic cells (BMDCs) were cultured *in vitro* and plated at 1 x 10⁶ cells/ml. The BMDCs were conditioned with oleamide or its family of analogues at a concentration of 25uM 1 hour prior to stimulation with 100ng/ml LPS (*E.Coli* serotype R515). After 24 hours, the supernatants were removed and analysed for levels of IL-12p40, IL-12p70, IL-23 and TNF-alpha using specific immunoassays. (n=9)
   The data sets below represent the following groups:
   Figs 4(A) Oleamide and oleamide analogues (Compounds 37-41 43, 69 and 104);

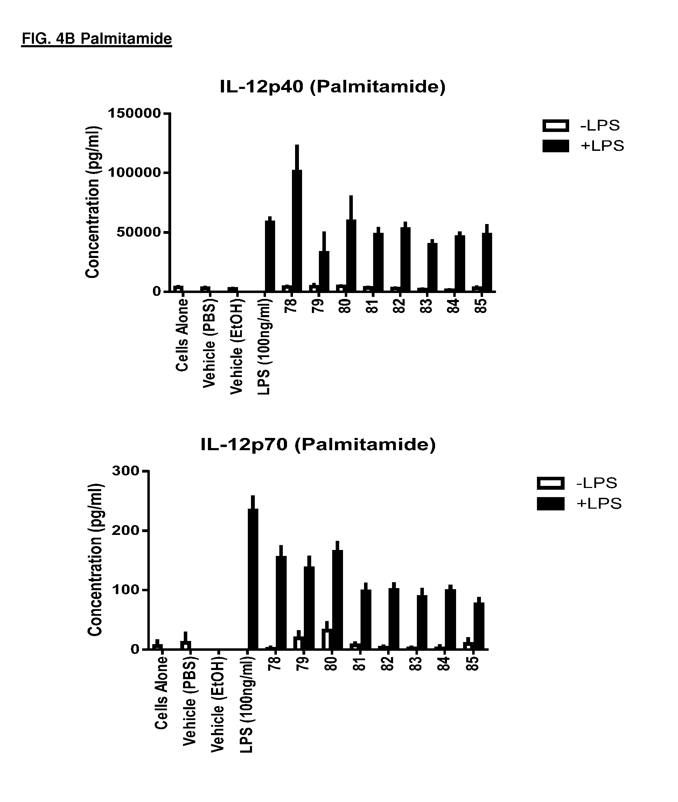

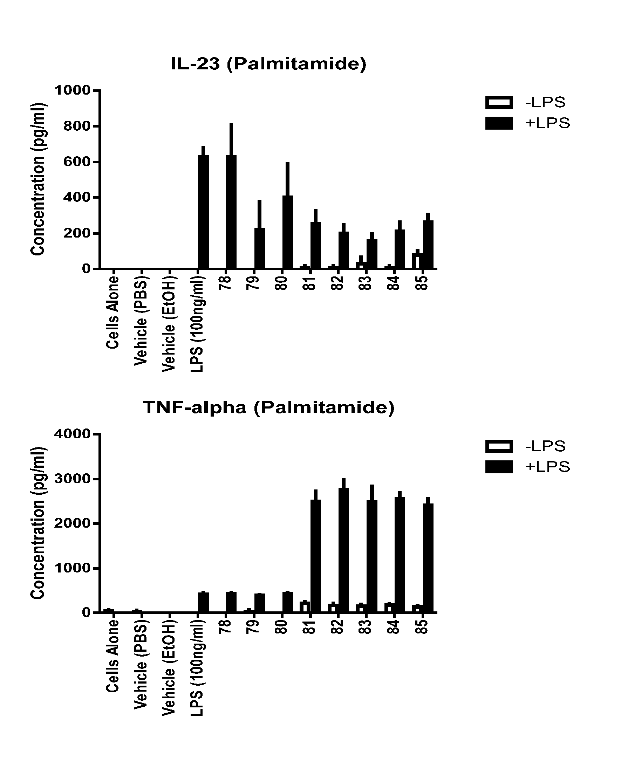

   Figs 4(B) Palmitamide and palmitamide analogues (Compounds 78-85);

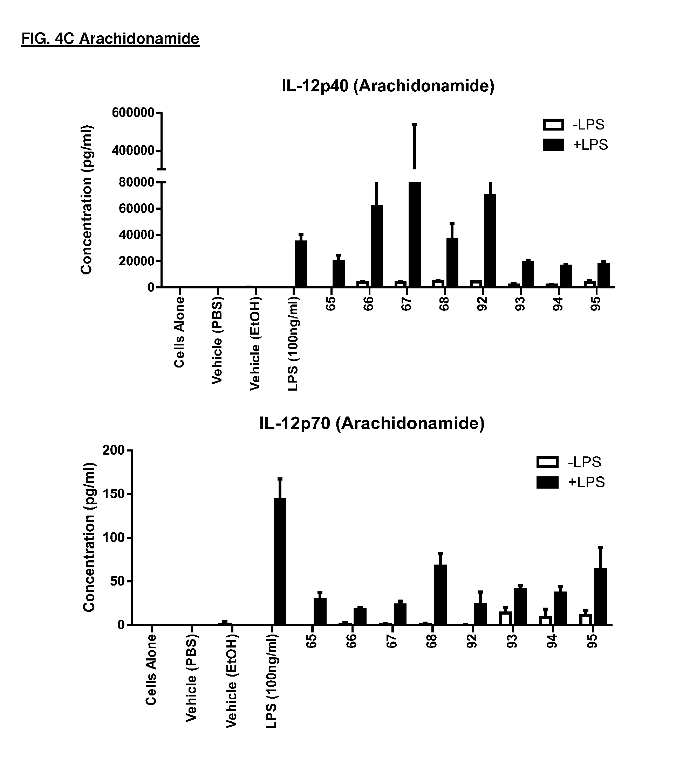

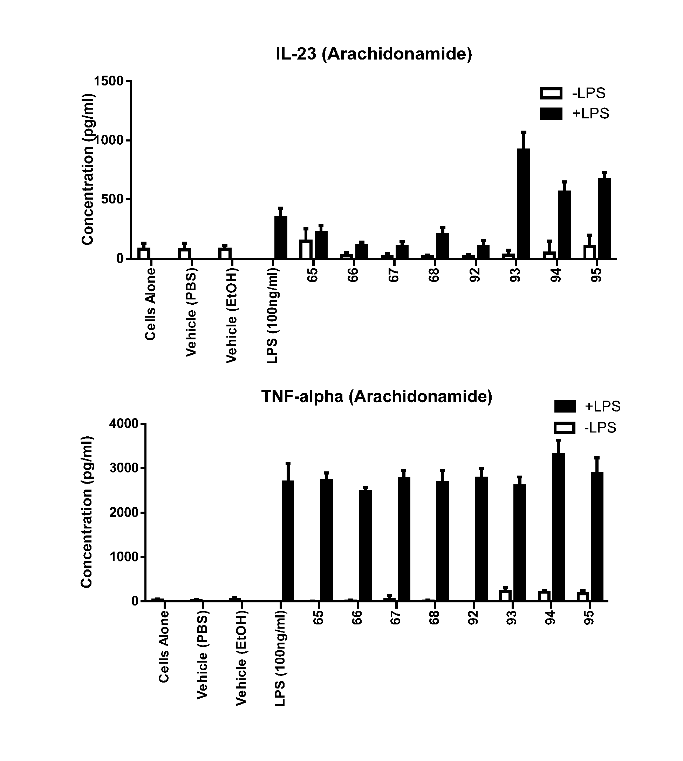

   Figs 4(C) Arachidonamide and arachidonamide analogues (Compounds 65-68 and 92-95),

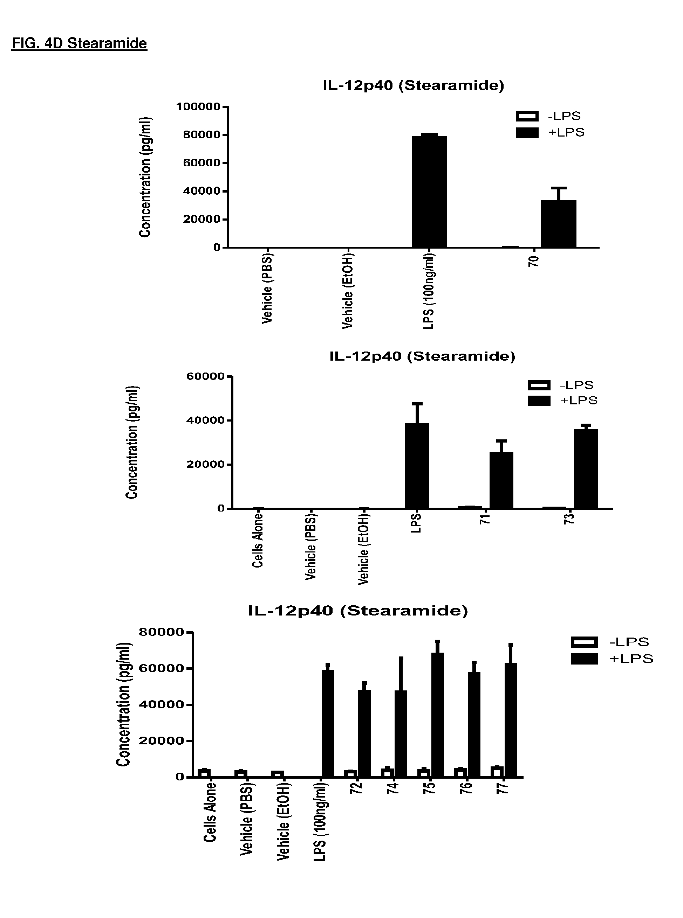

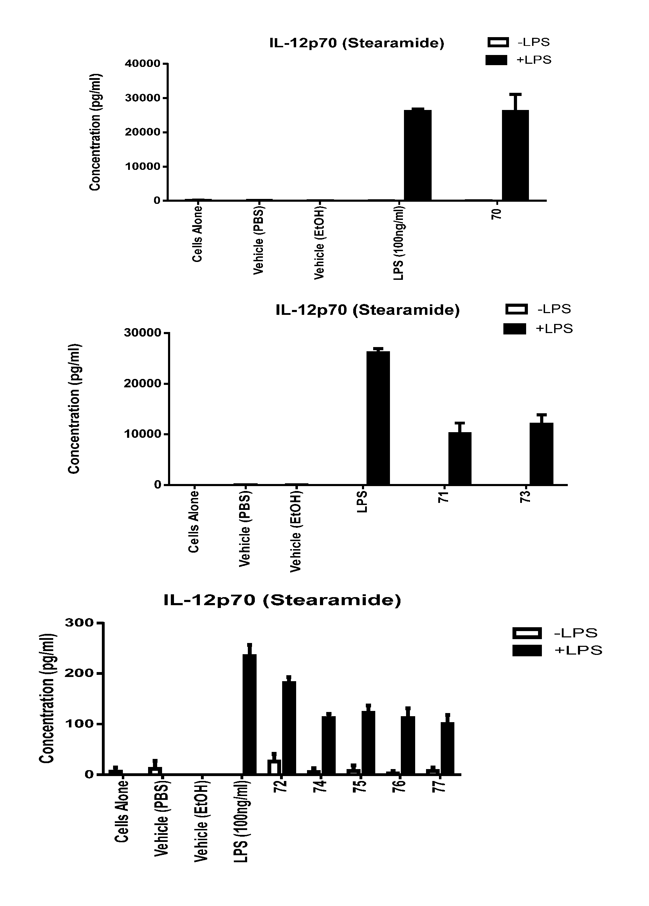

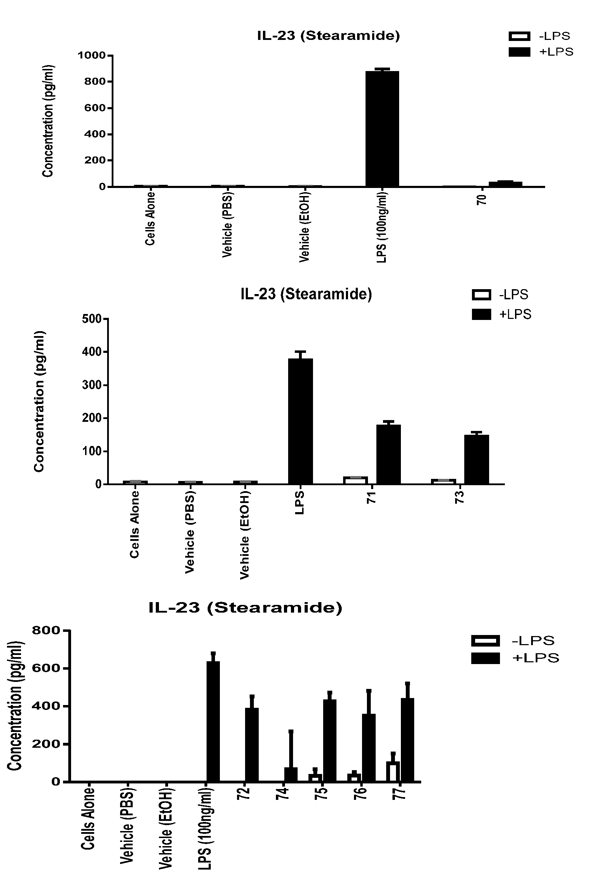

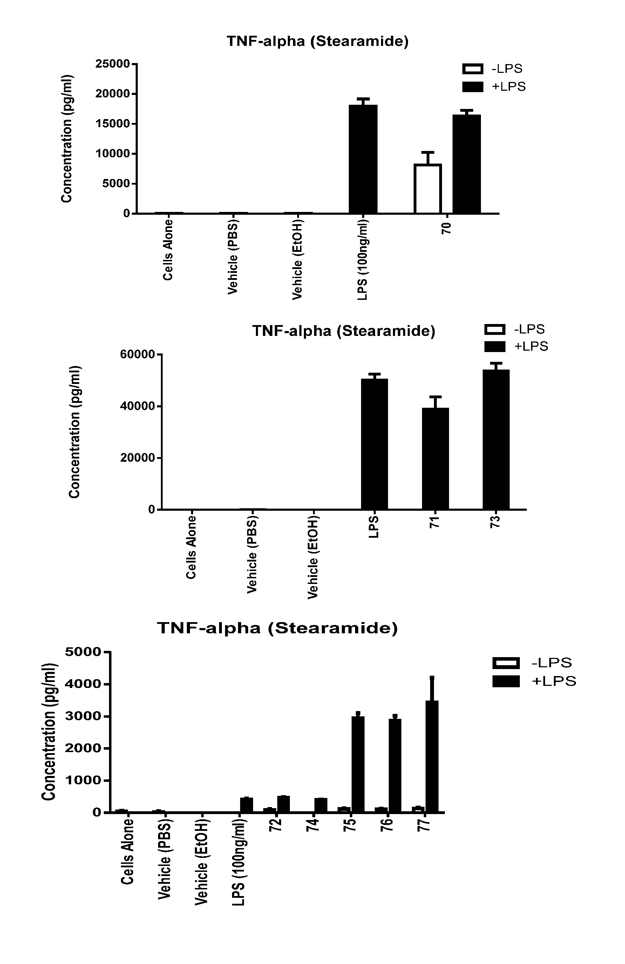

   Figs 4(D) Stearamide and stearamide analogues (Compounds 70-77); and

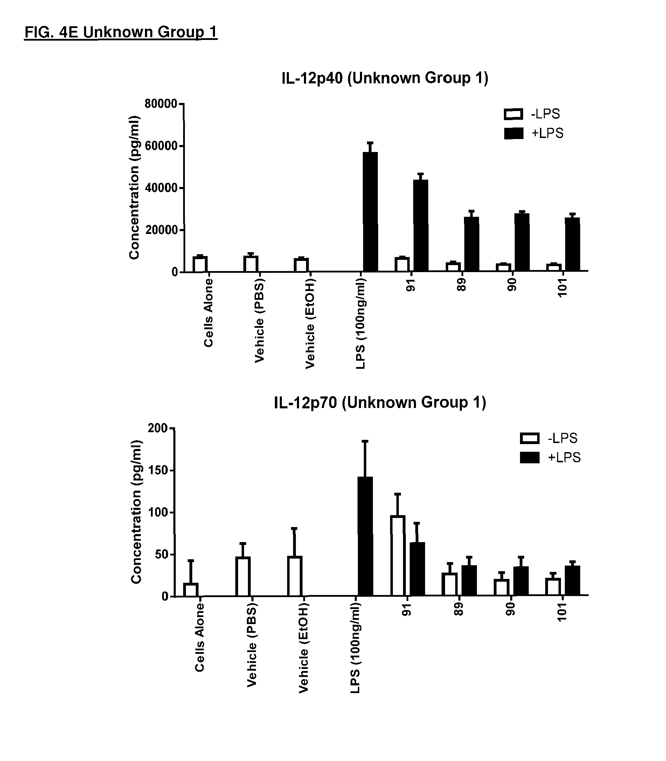

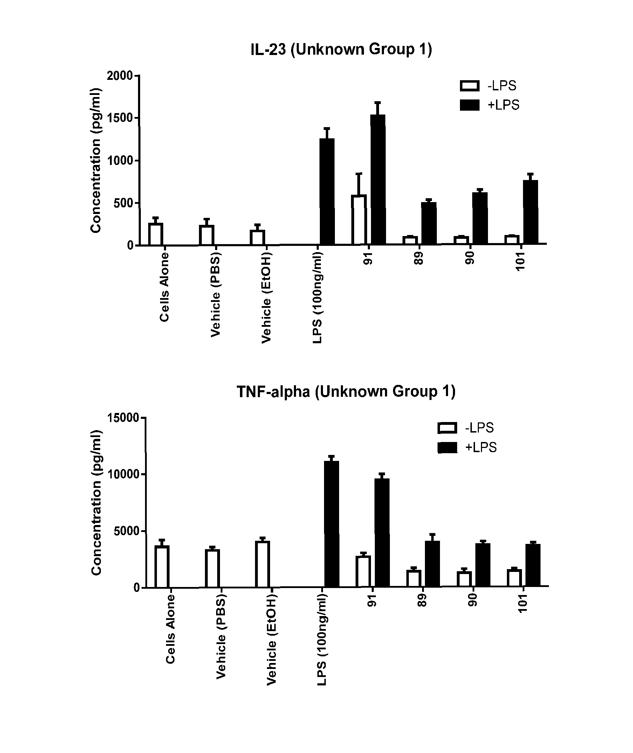

   Figs 4(E) Compounds 89-91 and 101;

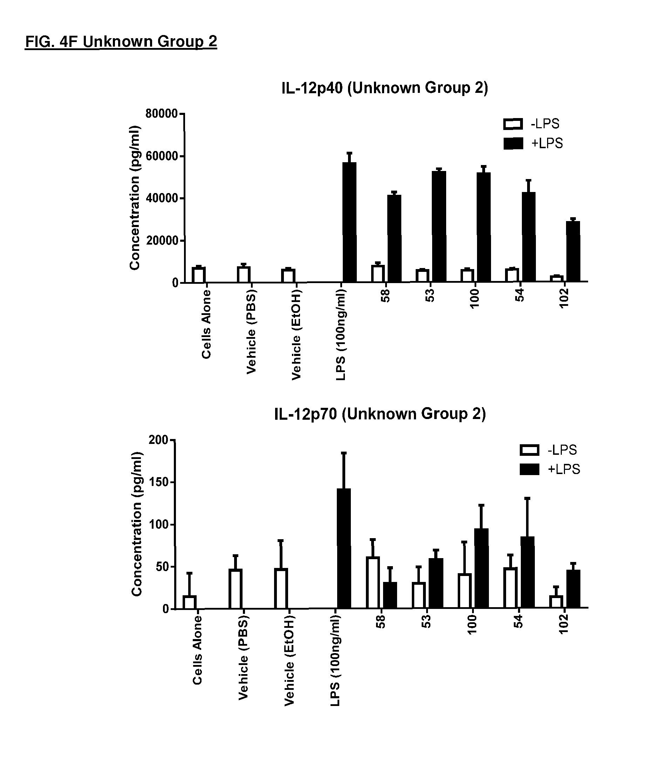

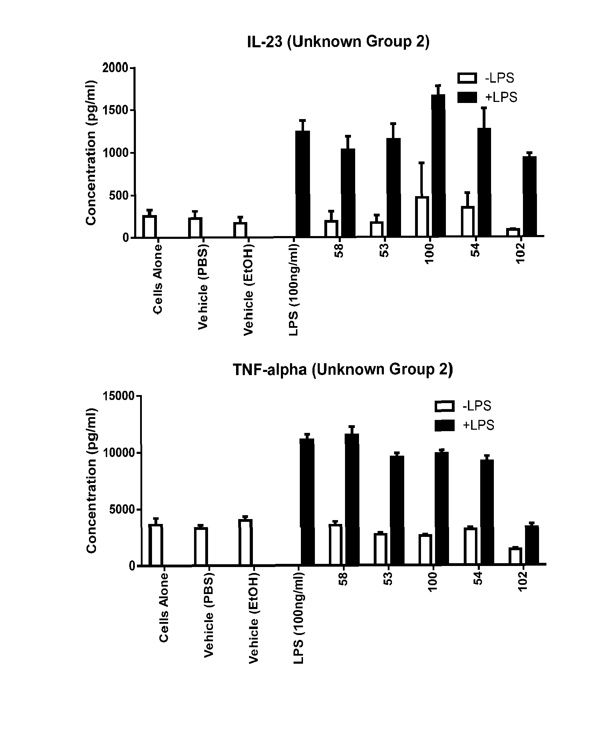

   Figs 4(F) Compounds 53, 54, 58, 100 and 102; and

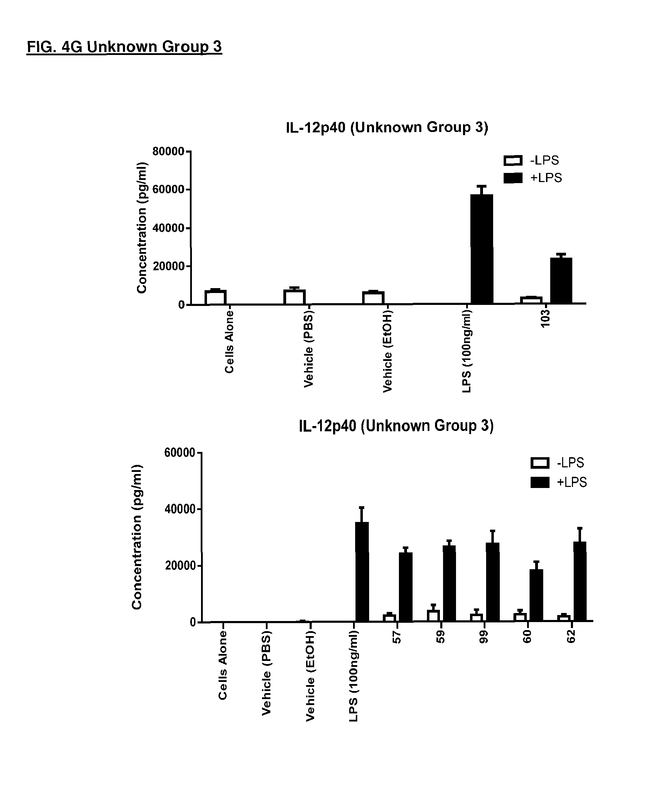

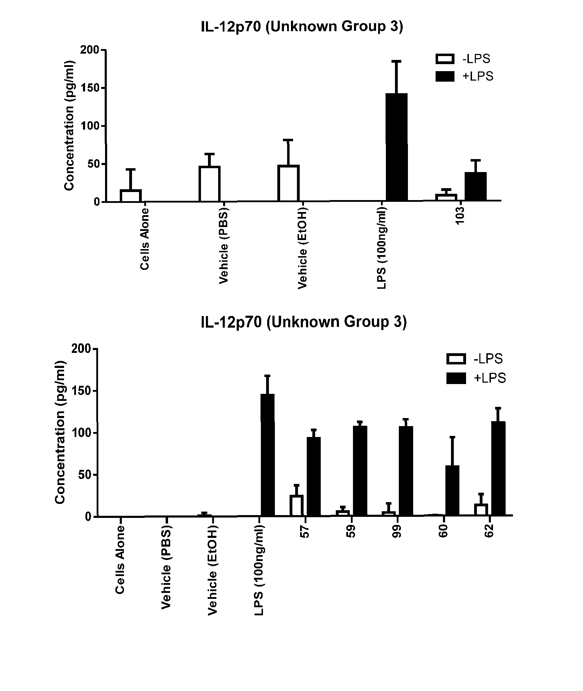

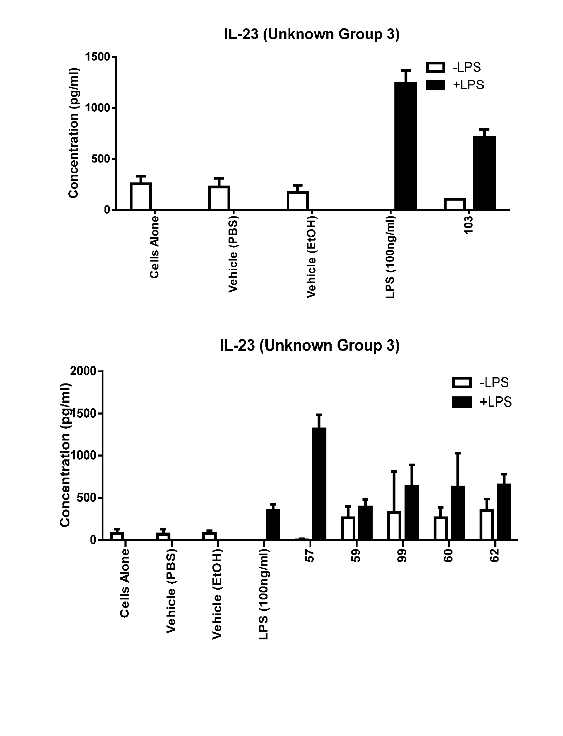

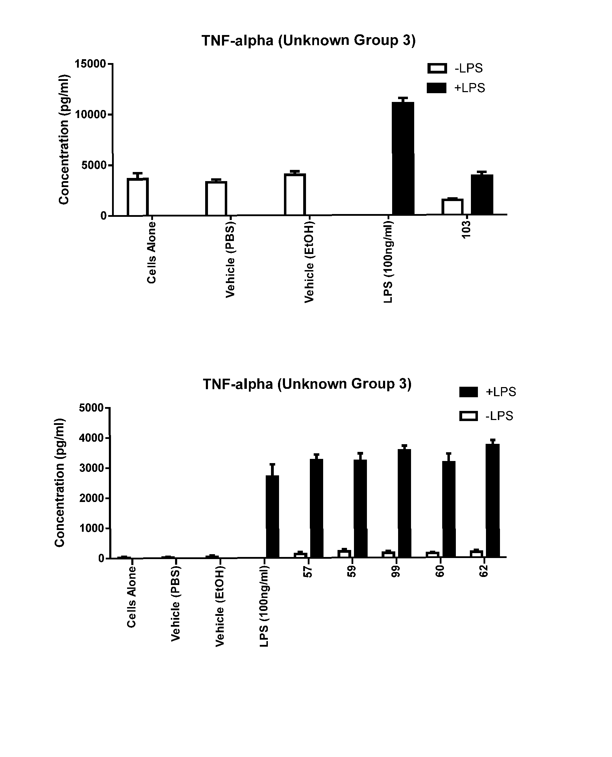

   Figs 4(G) Compounds 57, 59, 60, 62,99 and 103.
      Each histogram represents a different experiment.

### Detailed Description of the Drawings

The current invention has a specific target which only interferes with the TLR that is involved in disease and therefore leaves the normal TLR response to the majority of pathogens intact. The active of the present invention is a naturally occurring endogenous lipid which prevents phosphorylation of Mal thereby blocking responses associated with TLR4 and TLR2. Furthermore, it blocks Myd88-dependent responses (downstream of NF-κβ) only and leaves the Myd88-independent responses intact (downstream of IRF3). This means that some of the immune response is left intact such as the production of IFN-beta. Therefore, the specificity of the oleamide permits the specific blockage of the production of cytokines which are downstream of NF-κB such as IL-12 and IL-23, which are the known mediators of inflammatory disease. This minimises the impact of the treatment on the overall immune defence to pathogens.

A major obstacle of anti-cytokine therapy is the so-called non-responders who, depending on the study and the biological in use, can make up 28-50% of the respective patient population. Specific suppression of Mal may provide an important additional therapeutic approach aiming at the group of patients who failed conventional anti-inflammatory intervention.

This invention addresses a clinical problem. Given the link between both TLR2 and TLR4 and a number of inflammatory diseases, interfering with the Mal downstream of these TLRs is an attractive target for these diseases - these include rheumatoid arthritis, inflammatory bowel disease, atherosclerosis, multiple sclerosis, cardiovascular disease and diabetes. Therefore, this inhibitor can be used in the treatment of inflammatory diseases that are associated with overactivation/expression of TLR4, TLR2 or Mal. Furthermore, given that Mal is unique to these 2 TLRs, a significant advantage of targeting this adaptor protein is that the function of the other TLRs remain intact and therefore the patient can maintain the ability to respond normally to a wide range of pathogens.

### Definitions:

"Inflammatory disorder" means an immune-mediated inflammatory condition that affects humans and is characterised by dysregulated expression of one or more cytokines which are downstream of NFκB such as TNF-α, IL-12 and IL-23 and/or over-activation of Toll-like Receptor 4 (TLR4), Toll-like Receptor 2 (TLR2) or Myeloid differentiating protein 88 (Myd88) adaptor-like protein (Mal). In a preferred embodiment, the term means an immune-mediated inflammatory condition that affects humans and is characterised by dysregulated expression of one or more cytokines which are downstream of NF-κB such as TNF-α, IL-12, and IL-23 and over-activation of Toll-like Receptor 4 (TLR4), Toll-like Receptor 2 (TLR2) and Myeloid differentiating protein 88 (Myd88) adaptor-like protein (Mal).

"Peripheral" as applied to inflammatory disorders means an inflammatory disorder that is not mediated by cells of the nervous system, especially the central nervous system, in humans (i.e. a pathology that is not mediated by glial cells or neurons). The peripheral inflammatory disorder does not include inflammatory diseases of the brain or central nervous system. Examples of peripheral inflammatory disorders include skin inflammatory disorders, inflammatory disorders of the joints, inflammatory disorders of the cardiovascular system, certain autoimmune diseases, lung and airway inflammatory disorders, intestinal inflammatory disorders. Examples of skin inflammatory disorders include dermatitis, for example atopic dermatitis and contact dermatitis, acne vulgaris, and psoriasis. Examples of inflammatory disorders of the joints include rheumatoid arthritis. Examples of inflammatory disorders of the cardiovascular system are cardiovascular disease and atherosclerosis. Examples of autoimmune diseases include Type 1 diabetes, Graves disease, Guillain-barré disease, Lupus, Psoriatic arthritis, and Ulcerative colitis. Examples of lung and airway inflammatory disorders include asthma, cystic fibrosis, COPD, emphysema, and acute respiratory distress syndrome. Examples of intestinal inflammatory disorders include colitis and inflammatory bowel disease.

"Immunomodulatory fatty acid-based compound" means an immunomodulatory fatty acid, an immunomodulatory amide of a fatty acid (hereafter "fatty acid amide"), or an analogue of an amide of a fatty acid (hereafter "fatty acid amide analogue") that is immunomodulatory, "Immunomodulatory" means capable of decreasing expression of at least one pro-inflammatory cytokine selected from IL-12, IL-23 and TNF-α in the LPS cell model described below.

"Immunomodulatory fatty acid" means oleic acid, palmitoleic acid, stearic acid, arachidonic acid, linoleic acid and linolenic acid.

"Immunomodulatory fatty acid amide" means oleamide, palmitamide, arachidonamide, stearamide, palmitoleamide, linoleamide, and linolenamide.

"Oleamide" is primary amide, oleamide (9(Z)-Octadecenamide), which is an unsaturated fatty acid amide and shown in Fig. 1.

"Immunomodulatory analogue" as applied to oleamide refers to an analogue of oleamide in which the amide group is modified by one or more substituents and which is capable of decreasing expression of at least one pro-inflammatory cytokine selected from IL-12, IL23 and TNF-α in the LPS cell model described below. Examples include compounds 37, 38, 39, 40, 41, 43, 44, 69 and 104 of Table 1. Preferably, the immunomodulatory analogue is a potent immunomodulatory analogue.

"Immunomodulatory analogue" as applied to palmitamide refers to an analogue of palmitamide in which the amide group is modified by one or more substituents and which is capable of decreasing expression of at least one pro-inflammatory cytokine selected from IL-12, IL23 and TNF-α in the LPS cell model described below. Examples include compounds 79, 80, 81, 82, 83, 84 and 85 of Table 1. Preferably, the immunomodulatory analogue is a potent immunomodulatory analogue.

"Immunomodulatory analogue" as applied to arachidonamide refers to an analogue of arachidonamide in which the amide group is modified by one or more substituents and which is capable of decreasing expression of at least one pro-inflammatory cytokine selected from IL-12, IL23 and TNF-α in the LPS cell model described below. Examples include compounds 65, 66, 67, 68, 92, 93, 94, 95 of Table 1. Preferably, the immunomodulatory analogue is a potent immunomodulatory analogue.

"Immunomodulatory analogue" as applied to stearamide refers to an analogue of stearamide in which the amide group is modified by one or more substituents and which is capable of decreasing expression of at least one pro-inflammatory cytokine selected from IL-12, IL23 and TNF-α in the LPS cell model described below. Examples include compounds 70, 71, 72, 73, 74, 75, 76 and 77 of Table 1. Preferably, the immunomodulatory analogue is a potent immunomodulatory analogue.

"Immunomodulatory analogue" as applied to palmitoleamide refers to an analogue of palmitoleamide in which the amide group is modified by one or more substituents and which is capable of decreasing expression of at least one pro-inflammatory cytokine selected from IL-12, IL23 and TNF-α in the LPS cell model described below. Examples include compounds 89, 90, 91 and 101 of Table 1. Preferably, the immunomodulatory analogue is a potent immunomodulatory analogue.

"Immunomodulatory analogue" as applied to linoleamide refers to an analogue of linoleamide in which the amide group is modified by one or more substituents and which is capable of decreasing expression of at least one pro-inflammatory cytokine selected from IL-12, IL23 and TNF-α in the LPS cell model described below. Examples include compounds 53, 54, 58, 100 and 102 of Table 1. Preferably, the immunomodulatory analogue is a potent immunomodulatory analogue.

"Immunomodulatory analogue" as applied to linolenamide refers to an analogue of linolenamide in which the amide group is modified by one or more substituents and which is capable of decreasing expression of at least one pro-inflammatory cytokine selected from IL-12, IL23 and TNF-α in the LPS cell model described below. Examples include compounds 57, 59, 60, 62, 99 and 103 of Table 1. Preferably, the immunomodulatory analogue is a potent immunomodulatory analogue.

The term "potent" as applied to an immunomodulatory analogue means that the analogue is capable of decreasing expression of at least one pro-inflammatory cytokine selected from IL-12, IL-23 and TNF-α in the LPS cell model described below by at least 20% or preferably by at least 30%.

A further aspect of the invention relates to a pharmaceutical composition comprising an immunomodulatory fatty acid-based compound admixed with one or more pharmaceutically acceptable diluents, excipients or carriers. Even though the compounds of the present invention (including their pharmaceutically acceptable salts, esters and pharmaceutically acceptable solvates) can be administered alone, they will generally be administered in admixture with a pharmaceutical carrier, excipient or diluent, particularly for human therapy. The pharmaceutical compositions may be for human or animal usage in human and veterinary medicine.

Examples of such suitable excipients for the various different forms of pharmaceutical compositions described herein may be found in the "Handbook of Pharmaceutical Excipients, 2nd Edition, (1994), Edited by A Wade and PJ Weller. In particular, formulations for topical delivery are described in Topical drug delivery formulations edited by David Osborne and Antonio Aman, Taylor & Francis, the complete contents of which are incorporated herein by reference.
(ISBN 082478183X, 9780824781835)

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol, sorbitol and the like. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s).

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like.

Preservatives, stabilizers, dyes and even flavouring agents may be provided in the pharmaceutical composition. Examples of preservatives include sodium benzoate, sorbic acid and esters of p-hydroxybenzoic acid. Antioxidants and suspending agents may be also used.

The immunomodulatory fatty acid-based compound may be adapted for topical, oral, rectal, parenteral, intramuscular, intraperitoneal, intra-arterial, intrabronchial, subcutaneous, intradermal, intravenous, nasal, vaginal, buccal or sublingual routes of administration.

For oral administration, particular use is made of compressed tablets, pills, tablets, gellules, drops, and capsules. Preferably, these compositions contain from 1 to 250 mg and more preferably from 10-100 mg, of active ingredient per dose.

Other forms of administration comprise solutions or emulsions which may be injected intravenously, intra-arterial, subcutaneously, intradermally, intraperitoneally or intramuscularly, and which are prepared from sterile or sterilisable solutions. The pharmaceutical compositions of the present invention may also be in form of suppositories, vaginal rings, pessaries, suspensions, emulsions, lotions, ointments, creams, gels, sprays, solutions or dusting powders.

The composition of the invention may be formulated for topical delivery. Topical delivery generally means delivery to the skin, but can also mean delivery to a body lumen lined with epithelial cells, for example the lungs or airways, the gastrointestinal tract, the buccal cavity. In particular, formulations for topical delivery are described in Topical drug delivery formulations edited by David Osborne and Antonio Aman, Taylor & Francis, the complete contents of which are incorporated herein by reference. Compositions or formulations for delivery to the airways are described in O'Riordan et al (Respir Care, 2002, Nov. 47), EP2050437, WO2005023290, US2010098660, and US20070053845. Composition and formulations for delivering active agents to the iluem, especially the proximal iluem, include microparticles and microencapsulates where the active agent is encapsulated within a protecting matrix formed of polymer or dairy protein that is acid resistant but prone to dissolution in the more alkaline environment of the ileum. Examples of such delivery systems are described in EP1072600.2 and EP13171757.1.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

Injectable forms may contain between 10-1000 mg, preferably between 10-250 mg, of active ingredient per dose.

Compositions may be formulated in unit dosage form, i.e., in the form of discrete portions containing a unit dose, or a multiple or sub-unit of a unit dose.

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

Depending upon the need, the agent may be administered at a dose of from 0.01 to 30 mg/kg body weight, such as from 0.1 to 10 mg/kg, more preferably from 0.1 to 1 mg/kg body weight.

In an exemplary embodiment, one or more doses of 10 to 300 mg/day or more preferably, 10 to 150 mg/day, will be administered to the patient for the treatment of a peripheral inflammatory disorder.

In a particularly preferred embodiment, the methods and uses of the invention involve administration of an immunomodulatory fatty acid-based compound in combination with one or more other active agents, for example, existing anti-inflammatory drugs or pharmacological enhancers available on the market. In such cases, the compounds of the invention may be administered consecutively, simultaneously or sequentially with the one or more other active agents.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

### EXPERIMENTAL

### MATERIALS AND METHODS

### Preparation of Oleamide and its Analogues

All reactions were carried out under an argon atmosphere with dry solvents unless otherwise noted. All commercial reagents were used without further purification. Since palmitoleic acid (101), linoleic acid (102), linolenic acid (103) and oleic acid (104) were commercially available, they were not synthesised and were supplied by TCI Europe (www.trichemicals.com/en/eu) (101-103) and Sigma Aldrich (www.sigmaaldrich.com/ireland) (104). Thin-layer chromatography (TLC) analysis of reaction mixtures was performed using TLC Silica gel 60 F₂₅₄ plates and visualized under UV (λ=254 nm) or by staining with acidic vanillin solution followed by heating. Flash column chromatography was carried out using 40-63 micron (230-400 mesh) silica gel. NMR spectrum were recorded using Varian 300, 400 MHz spectrometer using CDCl₃ as the solvent at ambient temperature using tetramethylsilane (for 1H NMR (δ= 0 ppm) or residual CHCl₃ for 1H (δ= 7.26 ppm) or ¹³C NMR (δ= 77.16 ppm) as the internal standard. Coupling constants (J) are measured in hertz (Hz). Multiplicities are reported using the following abbreviations: s = singlet, d = doublet, t = triplet, q = quartet, m = multiplet. Exact mass ESI mass spectra (HRMS) were measured on a micromass LCT orthogonal time-of-flight mass spectrometer with leucine enkephalin (Tyr-Gly-Phe-Leu) as an internal lock mass. FT-IR spectra are reported interms of frequency of absorption (cm⁻¹).

### Synthesis of Oleamide (Ole)

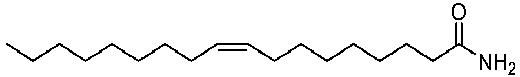

A solution of oleic acid (447 mg, 1.58 mmol) in dichloromethane in a 10 mL round bottomed flask was cooled to 0 °C before oxalyl chloride was added dropwise (0.4 mL, 4.74 mmol). The reaction was allowed to heat to 25 °C and left to stir for 4 h. The reaction mixture was concentrated under reduced pressure and again cooled to 0 °C. The concentrate was treated with saturated aq NH₄OH (5 mL) for 5 min. The mixture was partitioned between ethyl acetate (10 mL) and water (10 mL), before drying the organic layer over magnesium sulfate. The organic leyer was then concentrated under reduced pressure to yield the crude product. Purification by flash column chromatography (SiO₂, 3:2 pentane/ethyl acetate, increasing to 100% ethyl acetate) gave the title compound oleamide (302 mg, 46%) as a white solid.

R_{f} = 0.22 (Pentane/EtOAc, 3/1); IR (film) υₘₐₓ 1675, 1467 cm⁻¹ ; ¹H NMR (400 MHz, CDCl₃) δ 5.35 (m, 4H), 2.23 (t, *J* = 7.6 Hz, 2H), 2.01 (q, *J =* 6.1 Hz, 4H), 1.64 (m, 2H), 1.28 (m, 20H), 0.88 (t, *J* = 6.7 Hz, 3H); ¹³C NMR (101 MHz, CDCl₃) δ 206.80, 130.00, 129.70, 35.90, 31.90, 30.90, 29.70, 29.60, 29.50, 29.30, 29.20, 29.10, 27.20, 27.10, 25.50, 22.70, 14.10; HRMS: (ESI-TOF) calculated for C₁₈H₃₅NO [M - H⁺] 282.2797, found 294.2803.

### General Procedure for preparation of amides

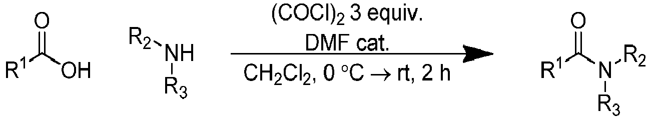

To the appropriate carboxylic acid (1 equiv.) in anhydrous dichloromethane 5 mL at 0 °C was added, drop wise, oxalyl chloride (3 equiv.) followed by 3 drops of dimethylformamide. Some effervescence was observed and the reaction was stirred under nitrogen while being allowed to warm to room temperature over 2 hours. The solvent was then removed *in vacuo* to produce an orange semi-solid. The semi-solid was dissolved in dichloromethane 5 mL and appropriate amine (10 equiv.) were added in a further 5 mL of dichloromethane and the reaction stirred for a further 5 minutes. The reaction mixture was then poured into a separating funnel and washed with water (2 x 10 mL), 10 % HCl (2 x 10 mL), NaHCO₃ solution (2 x 10 mL) and brine (10 mL). The organic layer was dried over MgSO₄ and the solvent reduced *in vacuo* to give the crude amide as a yellow oil, which was purified by column chromatography.

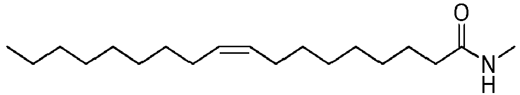

### N-methyloleamide (37)

Made according to the general procedure with methylamine, isolated as a white solid (247 mg, 78 % yield).

R_{f} = 0.27 (Pentane/EtOAc, 1/1); IR (film) υₘₐₓ 1639, 1467 cm⁻¹; ¹H NMR (400 MHz, CDCl₃) δ 5.42 (s, 1H), 5.32 (m, 2H), 2.78 (d, J = 4.8 Hz, 3H), 2.14 (m, 2H), 1.99 (m, 4H), 1.60 (m, 3H), 1.28 (m, 19H), 0.86 (m, 3H); ¹³C NMR (101 MHz, CDCl₃) δ 173.72, 129.95, 129.74, 36.73, 31.82, 29.71, 29.64, 29.42, 29.29, 29.28, 29.23, 29.10, 27.18, 27.14, 26.23, 25.74, 22.65, 14.08; HRMS: (ESI-TOF) calculated for C₁₉H₃₆NO [M - H⁺] 294.2797, found 294.2791.

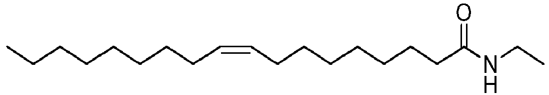

### N-ethyloleamide (38)

Made according to the general procedure with ethylamine, isolated as a white solid (368 mg, 85 % yield).

R_{f} =0.55 (Pentane/EtOAc, 1/1); IR (film) υₘₐₓ 1639, 1557 cm⁻¹; ¹H NMR (400 MHz, CDCl₃) δ 5.35 (m, 3H), 3.25 (m, 3H), 2.14 (m, 2H), 1.98 (m, 4H), 1.60 (m, 3H), 1.27 (m, 21H), 0.87 (m, 3H); ¹³C NMR (101 MHz, CDCl₃) δ 173.56, 129.86, 129.72, 41.77, 36.78, 31.33, 29.84, 29.77, 29.64, 29.28, 29.26, 29.10, 29.08, 27.45, 26.68, 25.54, 22.77, 22.54, 14.06, 11.45; HRMS: (ESI-TOF) calculated for C₂₀H₃₈NO [M - H⁺] 308.2953, found 308.2943.

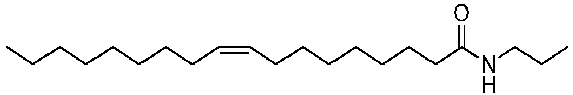

### N-propyloleamide (39)

Made according to the general procedure with *n*-propylamine, isolated as a white solid (394 mg, 86 % yield).

R_{f} = 0.77 (Pentane/EtOAc, 1/1); IR (film) υₘₐₓ 1639, 1555, 1468 cm⁻¹; ¹H NMR (400 MHz, CDCl₃) δ 5.33 (m, 3H), 3.20 (m, 2H), 2.13 (m, 2H), 1.99 (m, 4H), 1.61 (m, 2H), 1.50 (m, 3H), 1.28 (m, 19H), 0.88 (m, 6H); ¹³C NMR (101 MHz, CDCl₃) δ 172.92, 129.93, 129.78, 41.13, 36.97, 31.84, 29.76, 29.61, 29.74, 29.30, 29.28, 29.26, 29.23, 29.11, 27.19, 27.14, 25.80, 22.94, 22.63, 14.05, 11.32; HRMS: (ESI-TOF) calculated for C₂₁H₄₀NO [M - H⁺] 322.3110, found 322.3101.

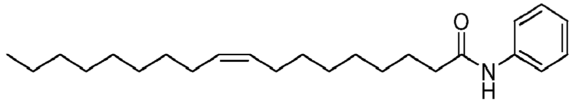

### N-phenyloleamide (40)

Made according to the general procedure with aniline, isolated as a white solid (340 mg, 68 % yield).

R_{f} = 0.12 (Pentane/EtOAc, 1/1); IR (film) υₘₐₓ 1652, 1602, 1466 cm⁻¹ ; ¹H NMR (400 MHz, CDCl₃) δ 7.49 (d, *J* = 7.9 Hz, 2H), 7.30 (m, 2H), 7.08 (m, 2H), 5.33 (m, 2H), 2.33 (t, *J* = 7.6 Hz, 2H), 2.00 (m, 4H), 1.71 (m, 2H), 1.27 (m, 20H), 0.86 (m, 3H); ¹³C NMR (101 MHz, CDCl₃) δ 129.97, 129.68, 128.93, 124.08, 119.67, 37.89, 31.87, 29.74, 29.68, 29.67, 29.50, 29.49, 29.29, 29.28, 29.24, 29.21, 29.09, 27.19, 27.13, 25.58, 22.65, 14.08; HRMS: (ESI-TOF) calculated for C₂₄H₄₀NO [M + H⁺] 358.5805, found 358.5802.

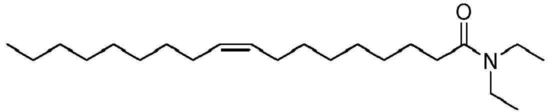

### N,N-diethyloleamide (41)

Made according to the general procedure with diethylamine, isolated as a clear oil (453 mg, 79% yield).

R_{f} = 0.61 (Pentane/EtOAc, 1/1); IR (film) υₘₐₓ 1641, 1462, 1431 cm⁻¹; ¹H NMR (400 MHz, CDCl₃) δ 5.32 (m, 2H), 3.35 (q, *J* = 7.1 Hz, 2H), 3.28 (q, *J* = 7.1 , 2H), 2.26 (m, 2H), 1.99 (m, 4H), 1.62 (m, 3H), 1.27 (m, 19H), 1.15 (t, *J* = 7.1 Hz, 3H), 1.08 (t, *J* = 7.1 Hz, 3H), 0.85 (m, 3H); ¹³C NMR (101 MHz, CDCl₃) δ 172.21, 129.97, 129.72, 41.94, 39.98, 33.15, 31.86, 29.73, 29.70, 29.49, 29.36, 29.29, 29.28, 29.15, 27.18, 27.16, 25.47, 22.64, 14.37, 14.07, 13.09; HRMS: (ESI-TOF) calculated for C₂₂H₄₃NONa [M + Na⁺] 360.3242, found 360.3247.

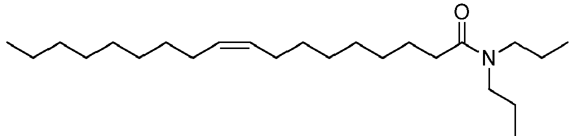

### N,N-dipropyloleamide (43)

Made according to the general procedure with *n*,*n*-dipropylamine, isolated as a clear oil (455 mg, 89% yield).

R_{f} = 0.80 (Pentane/EtOAc, 1/1); IR (film) υₘₐₓ 1646, 1465, 1424 cm⁻¹ ; ¹H NMR (400 MHz, CDCl₃) δ 5.33 (m, 2H), 3.25 (m, 2H), 3.16 (m, 2H), 2.25 (m, 2H), 1.99 (m, 4H), 1.56 (m, 6H), 1.29 (m, 22H), 0.88 (m, 7H); ¹³C NMR (101 MHz, CDCl₃) δ 172.69, 129.90, 129.77, 49.60, 47.42, 33.15, 31.86, 29.73, 29.71, 29.48, 29.37, 29.28, 29.15, 27.18, 25.52, 22.65, 22.30, 20.95, 14.06, 11.37, 11.22; HRMS: (ESI-TOF) calculated for C₂₄H₄₇NONa [M + Na⁺] 388.3555, found 388.3553.

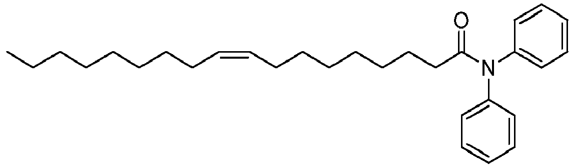

### N,N-diphenyloleamide (44)

Made according to the general procedure with diphenylamine, isolated as a clear oil (327 mg, 54 % yield).

R_{f} = 0.58 (Pentane/EtOAc, 25/1); IR (film) υₘₐₓ 1661, 1499, 1444 cm⁻¹; ¹H NMR (400 MHz, CDCl₃) δ 7.25 (m, 4H), 7.06 (m, 4H), 6.92 (m, 2H), 5.28 (m, 1H), 2.34 (t, *J* = 7.5 Hz, 1H), 2.03 (m, 3H), 1.58 (m, 1H), 1.18 (m, 25H), 0.82 (m, 2H); ¹³C NMR (101 MHz, CDCl₃) δ 178.91, 143.14, 129.99, 129.61, 129.29, 120.93, 117.90, 87.01, 33.87, 31.91, 29.74, 29.65, 29.50, 29.30, 29.11, 29.04, 29.00, 27.19, 27.12, 24.62, 22.65, 14.05; HRMS: (ESI-TOF) calculated for C₃₀H₄₄NO [M + H⁺] 434.6765, found 434.6762.

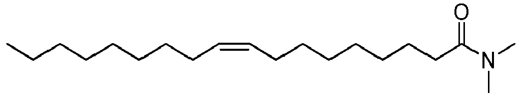

### N,N-dimethyloleamide (69)

Made according to the general procedure with dimethylamine, isolated as a clear oil (243 mg, 82 % yield).

R_{f} = 0.39 (Pentane/EtOAc, 1/1); IR (film) υₘₐₓ 1641, 1461, 1431 cm⁻¹; ¹H NMR (400 MHz, CDCl₃) δ 5.32 (m, 2H), 2.98 (s, 3H), 2.92 (s, 3H), 2.00 (m, 4H), 1.60 (m, 2H), 1.27 (m, 20H), 0.82 (m, 3H); ¹³C NMR (101 MHz, CDCl₃) δ 173.26, 129.91, 129.77, 37.23, 35.32, 33.41, 31.87, 29.74, 29.70, 29.49, 29.47, 29.34, 29.29, 29.14, 27.18, 27.15, 25.16, 22.65, 14.08; ; HRMS: (ESI-TOF) calculated for C₂₀H₄₀NO [M + H⁺] 310.3110, found 310.3123.

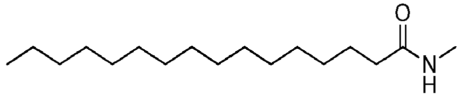

### N-methylpalmitamide (78)

Made according to the general procedure with methylamine, isolated as a white solid (226 mg, 77 % yield).

R_{f} = 0.32 (Pentane/EtOAc, 1/1); IR (film) υₘₐₓ 1640 cm⁻¹; ¹H NMR (400 MHz, CDCl₃) δ 2.78 (d, J = 4.8 Hz, 3H), 2.14 (m, 2H), 1.60 (m, 2H), 1.23 (m, 25H), 0.85 (m, 3H); ¹³C NMR (101 MHz, CDCl₃) δ 173.26, 36.66, 31.85, 29.66, 29.65, 29.63, 29.62, 29.61, 29.57, 29.45, 29.33, 29.32, 29.31, 26.28, 25.75, 22.66, 14.06; HRMS: (ESI-TOF) calculated for C₁₇H₃₅NONa [M + Na⁺] 292.2616, found 292.2622.

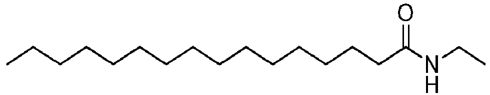

### N-ethylpalmitamide (79)

Made according to the general procedure with ethylamine, isolated as a white solid (259 mg, 84 % yield).

R_{f} = 0.41 (Pentane/EtOAc, 1/1); IR (film) υₘₐₓ 1639 cm⁻¹ ; ¹H NMR (400 MHz, CDCl₃) δ 3.27 (m, 3H), 2.12 (m, 2H), 1.60 (m, 2H), 1.27 (m, 24H, 1.12 (t, *J* = 7.3 Hz, 3H), 0.86 (m, 3H); ¹³C NMR (101 MHz, CDCl₃) δ 172.95, 36.85, 34.22, 31.88, 29.66, 29.65, 29.64, 29.62, 29.58, 28.57, 29.46, 29.33, 29.32, 29.28, 25.77, 22.64, 14.92, 14.08; HRMS: (ESI-TOF) calculated for C₁₈H₃₇NONa [M + Na⁺] 306.2773, found 306.2781.

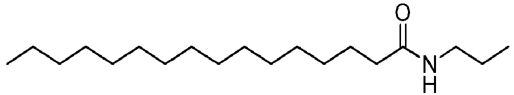

### N-propylpalmitamide (80)

Made according to the general procedure with *n*-propylamine, isolated as a white solid (275 mg, 85 % yield).

R_{f} = 0.50 (Pentane/EtOAc, 1/1); IR (film) υₘₐₓ 1641cm⁻¹; ¹H NMR (400 MHz, CDCl₃) δ 5.42 (s, 1H), 3.22 (m, 3H), 2.12 (t, *J =* 7.1 Hz, 3H), 1.62 (m, 3H), 1.49 (m, 3H), 1.23 (17H), 0.87 (m, 9H); ¹³C NMR (101 MHz, CDCl₃) δ 172.82, 42.34, 37.88, 32.45, 29.68, 29.67, 29.64, 29.61, 29.60, 29.59, 29.56, 29.46, 29.29, 29.27, 25.64, 22.78, 22.66, 14.08, 11.21; HRMS: (ESI-TOF) calculated for C₁₉H₃₉NONa [M + Na⁺] 320.2929, found 320.2937.

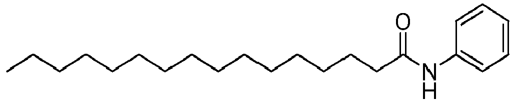

### N-phenylpalmitamide (81)

Made according to the general procedure with aniline, isolated as a white solid (260 mg, 72 % yield).

R_{f} = 0.20 (Pentane/EtOAc, 1/1); IR (film) υₘₐₓ 1650 cm⁻¹; ¹H NMR (400 MHz, CDCl₃) δ 7.49 (d, *J* = 7.9 Hz, 2H), 7.30 (t, app. *J* = 7.9 Hz, 2H), 7.08 (m, 1H), 2.33 (t, *J* = 7.6 Hz, 2H), 1.72 (m, 2H), 1.29 (m, 25H), 0.86 (m, 3H); ¹³C NMR (101 MHz, CDCl₃) δ 171.07, 128.98, 128.93, 124.12, 119.64, 37.84, 31.85, 29.68, 29.66, 29.65, 29.63, 29.61, 29.58, 29.44, 29.34, 29.32, 29.24, 25.59, 22.69, 14.07; HRMS: (ESI-TOF) calculated for C₂₂H₃₇NONa [M + Na⁺] 354.2773, found 354.2785.

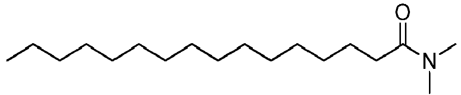

### N,N-dimethylpalmitamide (82)

Made according to the general procedure with dimethylamine, isolated as a white solid (265 mg, 86 % yield).

R_{f} = 0.44 (Pentane/EtOAc, 1/1); IR (film) υₘₐₓ 1640 cm⁻¹; ¹H NMR (400 MHz, CDCl₃) δ 2.99 (s, 3H), 2.93 (s, 3H), 2.28 (m, 2H), 1.62 (m, 2H), 1.26 (m, 24H), 0.86 (m, 3H); ¹³C NMR (101 MHz, CDCl₃) δ 173.65, 37.25, 35.29, 33.39, 31.89, 29.66, 29.65, 29.64, 29.63, 29.62, 29.60, 29.50, 29.44, 29.33, 25.18, 22.68, 14.08; HRMS: (ESI-TOF) calculated for C₁₈H₃₇NONa [M + Na⁺] 306.2773, found 306.2767.

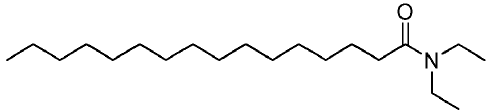

### N,N-diethylpalmitamide (83)

Made according to the general procedure with diethylamine, isolated as a white solid (292 mg, 86 % yield).

R_{f} = 0.43 (Pentane/EtOAc, 2/1); IR (film) υₘₐₓ 1639 cm⁻¹; ¹H NMR (400 MHz, CDCl₃) δ 3.31 (dq, *J* = 25 Hz, 7.1 Hz, 4H), 2.26 (m, 2H), 1.62 (m, 2H), 1.22 (m, 24H), 1.12 (dt, *J* = 25Hz, 7.1Hz, 6H), 0.86 (m, 3H); ¹³C NMR (101 MHz, CDCl₃) δ 172.16, 41.90, 39.97, 33.22, 31.84, 29.65, 29.63, 29.62, 29.60, 29.53, 29.50, 29.47, 29.33, 25.52, 22.66, 14.36, 14.05, 13.07; HRMS: (ESI-TOF) calculated for C₂₀H₄₁NONa [M + Na⁺] 334.3086, found 334.3089.

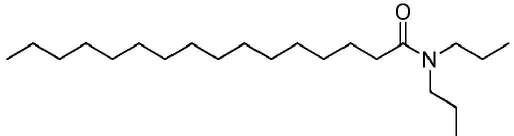

### N,N-dipropylpalmitamide (84)

Made according to the general procedure with *n*,*n*-dipropylamine, isolated as a white solid (325 mg, 88 % yield).

R_{f} = 0.53 (Pentane/EtOAc, 2/1); IR (film) υₘₐₓ 1638 cm⁻¹; NMR (400 MHz, CDCl₃) δ 3.25 (m, 2H), 3.16 (m, 2H), 2.26 (m, 2H), 1.56 (m, 7H), 1.28 (m, 24H), 0.87 (m, 8H); ¹³C NMR (101 MHz, CDCl₃) δ 172.56, 49.66, 47.43, 33.21, 31.85, 29.65, 29.63, 29.62, 29.60, 29.51, 29.47, 29.32, 25.51, 22.59, 22.26, 21.05, 14.13, 11.47, 11.21; HRMS:

(ESI-TOF) calculated for C₂₂H₄₅NONa [M + Na⁺] 362.3399, found 362.3405.

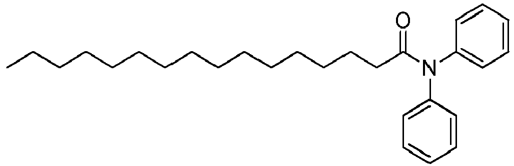

### N,N-diphenylpalmitamide (85)

Made according to the general procedure with diphenylamine, isolated as a white solid (257 mg, 58 % yield).

R_{f} =0.61 (Pentane/EtOAc, 25/1); IR (film) υₘₐₓ 1650 cm⁻¹; ¹H NMR (400 MHz, CDCl₃) δ 7.26 (m, 4H), 7.08 (m, 4H), 6.92 (m, 2H), 2.24 (m, 2H), 1.64 (m, 2H), 1.23 (m, 24H), 0.87 (m, 3H); ¹³C NMR (101 MHz, CDCl₃) δ 173.10, 143.26, 129.79, 121.25, 117.88, 38.44, 35.30, 31.89, 29.67, 29.65, 29.63, 29.62, 29.58, 29.44, 29.34, 29.32, 29.22, 25.59, 22.67, 14.13; HRMS: (ESI-TOF) calculated for C₂₈H₄₁NONa [M + Na⁺] 430.3086, found 430.3093.

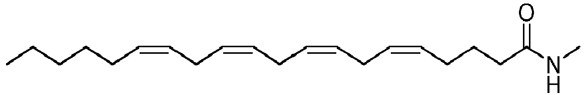

### N-methylarachidonamide (65)

Made according to the general procedure with methylamine, isolated as a clear oil (453 mg, 87 % yield).

R_{f} = 0.12 (Pentane/EtOAc, 1/1); IR (film) υₘₐₓ 1638, 1466, 1432 cm⁻¹; ¹H NMR (400 MHz, CDCl₃) δ 5.34 (m, 8H), 2.78 (m, 9H), 2.08 (m, 6H), 1.69 (m, 3H), 1.28 (m, 6H), 0.86 (m, 3H); ¹³C NMR (101 MHz, CDCl₃) δ 173.26, 130.49, 129.10, 128.69, 128.55, 128.17, 128.13, 127.80, 127.47, 35.98, 31.44, 29.26, 27.22, 26.84, 26.61, 26.30, 26.23, 25.60, 25.47, 22.54, 14.01; HRMS: (ESI-TOF) calculated for C₂₁H₃₆NO [M + H⁺] 318.2797, found 318.2792.

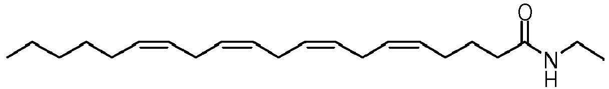

### N-ethylarachidonamide (66)

Made according to the general procedure with ethylamine, isolated as a clear oil (413 mg, 76 % yield).

R_{f} = 0.45 (Pentane/EtOAc, 1/1); IR (film) υₘₐₓ 1639, 1465, 1435 cm⁻¹; NMR (400 MHz, CDCl₃) δ 5.36 (m, 8H), 3.27 (m, 2H), 2.79 (m, 6H), 2.09 (m, 6H), 1.70 (m, 3H), 1.31 (m, 6H), 1.12 (m, 3H), 0.86 (m, 3H); ¹³C NMR (101 MHz, CDCl₃) δ 172.55, 130.51, 129.15, 128.69, 128.56, 128.18, 128.14, 127.81, 127.47, 36.15, 34.30, 32.65, 31.55, 29.37, 27.16, 26.79, 26.62, 25.62, 25.53, 22.55, 14.91, 14.04; HRMS: (ESI-TOF) calculated for C₂₂H₃₈NO [M + H⁺] 332.2953, found 332.2965.

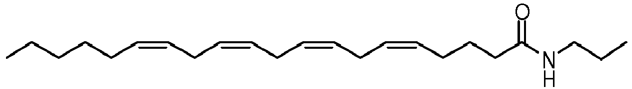

### N-propylarachidonamide (67)

Made according to the general procedure with *n*-propylamine, isolated as a clear oil (481 mg, 85 % yield).

R_{f} = 0.48 (Pentane/EtOAc, 1/1); IR (film) υₘₐₓ 1640, 1465, 1432 cm⁻¹; ¹H NMR (400 MHz, CDCl₃) δ 5.36 (m, 8H), 3.23 (m, 2H), 2.79 (m, 6H), 2.15 (m, 2H), 2.07 (m, 4H), 1.67 (m, 2H), 1.54 (m, 3H), 1.29 (m, 6H), 0.91 (m, 6H); ¹³C NMR (101 MHz, CDCl₃) δ 172.56, 130.49, 129.16, 128.68, 128.57, 128.18, 128.15, 127.82, 127.47, 41.11, 36.15, 35.96, 31.51, 29.29, 27.91, 27.20, 26.66, 25.60, 25.52, 22.98, 22.50, 14.04, 11.29; HRMS: (ESI-TOF) calculated for C₂₃H₃₉NO [M + H⁺] 346.3110, found 346.3120.

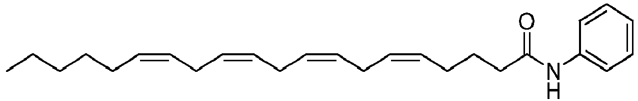

### N-phenylarachidonamide (68)

Made according to the general procedure with aniline, isolated as a clear oil (522 mg, 84 % yield).

R_{f} = 0.40 (Pentane/EtOAc, 1/1); IR (film) υₘₐₓ 1651, 1460 cm⁻¹ ; ¹H NMR (400 MHz, CDCl₃) δ 7.36 (m, 2H), 7.25 (m, 3H), 5.29 (m, 8H), 2.28 (t, *J* = 7.2 Hz, 2H), 2.04 (m, 4H), 1.60 (m, 4H), 1.27 (m, 11H), 0.86 (m, 3H); ¹³C NMR (101 MHz, CDCl₃) δ 172.86, 143.25, 140.21, 132.86, 130.79, 129.77, 128.57, 128.44, 128.01, 127.46, 127.32, 121.94, 117.82, 34.82, 31.98, 29.77, 27.56, 26.89, 25.46, 25.39, 25.32, 25.28, 22.11, 14.08; ; HRMS: (ESI-TOF) calculated for C₂₆H₃₇NONa [M + Na⁺] 402.2773, found 402.2784.

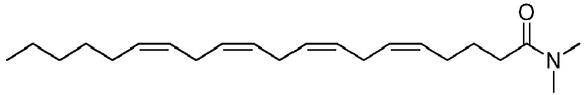

### N,N-dimethylarachidonamide (92)

Made according to the general procedure with dimethylamine, isolated as a clear oil (403 mg, 76 % yield).

R_{f} = 0.34 (Pentane/EtOAc, 1/1); IR (film) υₘₐₓ 1638, 1459 cm⁻¹; ¹H NMR (400 MHz, CDCl₃) δ 5.36 (m, 8H), 2.98 (s, 3H), 2.92 (s, 3H), 2.80 (m, 6H), 2.30 (m, 2H), 2.08 (m, 4H), 1.71 (m, 2H), 1.31 (m, 6H), 0.87 (m, 3H); ¹³C NMR (101 MHz, CDCl₃) δ 172.95, 130.46, 129.44, 128.57, 128.52, 128.23, 128.10, 127.85, 127.50, 37.19, 35.32, 32.64, 31.53, 29.32, 27.27, 26.81, 26.74, 25.64, 24.88, 22.50, 14.04; HRMS: (ESI-TOF) calculated for C₂₂H₃₈NO [M + H⁺] 332.2953, found 332.2961.

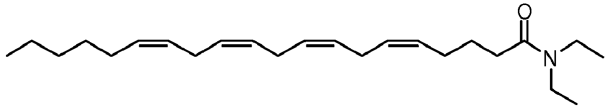

### N,N-diethylarachidonamide (93)

Made according to the general procedure with diethylamine, isolated as a clear oil (483 mg, 84 % yield).

R_{f} = 0.46 (Pentane/EtOAc, 1/1); IR (film) υₘₐₓ 1641, 1459 cm⁻¹ ; ¹H NMR (400 MHz, CDCl₃) δ 5.35 (m, 8H), 3.36 (q, *J* = 7.1 Hz, 2H), 3.27 (q, *J* = 7.1 Hz, 2H), 2.81 (m, 6H), 2.28 (m, 2H), 2.07 (m, 4H), 1.72 (p, app., *J* = 7.4 Hz, 2H), 1.28 (m, 6H), 1.12 (m, 6H), 0.87 (m, 3H); ¹³C NMR (101 MHz, CDCl₃) δ 172.16, 130.46, 129.54, 128.52, 128.50, 128.25, 128.08, 127.85, 127.48, 41.90, 39.97, 32.39, 31.52, 29.24, 27.20, 26.86, 25.63, 25.21, 22.60, 14.36, 14.03, 13.10; HRMS: (ESI-TOF) calculated for C₂₄H₄₂NO [M + H⁺] 360.3266, found 360.3272.

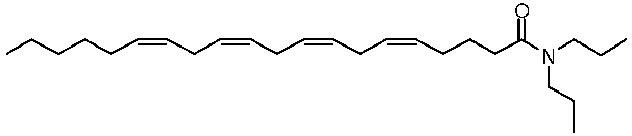

### N,N-dipropylarachidonamide (94)

Made according to the general procedure with *n*,*n*-dipropylamine, isolated as a clear oil (522 mg, 89 % yield).

R_{f} = 0.60 (Pentane/EtOAc, 1/1); IR (film) υₘₐₓ 1639, 1460 cm⁻¹ ; ¹H NMR (400 MHz, CDCl₃) δ 5.35 (m, 8H), 3.25 (m, 2H), 3.16 (m, 2H), 2.81 (m, 6H), 2.28 (t, *J* = 7.6 Hz, 2H), 2.07 (m, 4H), 1.72 (m, 2H), 1.54 (m, 4H), 1.30 (m, 6H), 0.88 (m, 9H); ¹³C NMR (101 MHz, CDCl₃) δ 172.28, 130.46, 129.54, 128.52, 128.47, 128.25, 128.09, 127.84, 127.51, 49.61, 47.43, 32.45, 31.45, 29.26, 27.17, 26.82, 25.61, 25.24, 22.52, 22.29, 20.95, 14.10, 11.39; HRMS: (ESI-TOF) calculated for C₂₆H₄₆NO [M + H⁺] 388.3579, found 388.3579.

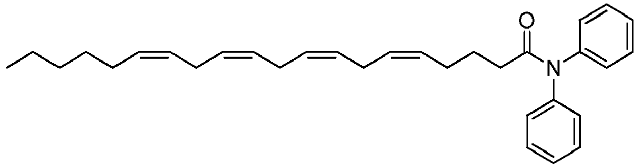

### N,N-diphenylarachidonamide (95)

Made according to the general procedure with diphenylamine, isolated as a clear oil (379 mg, 52 % yield).

R_{f} = 0.56 (Pentane/EtOAc, 25/1); IR (film) υₘₐₓ 1655, 1466 cm⁻¹; ¹H NMR (400 MHz, CDCl₃) δ 7.37 (m, 2H), 7.20 (m, 8H), 5.32 (m, 8H), 2.79 (m, 4H), 2.72 (m, 2H), 2.25 (t, *J* = 7.5 Hz, 2H), 2.02 (m, 4H), 1.72 (m, 2H), 1.30 (m, 6H), 0.87 (m, 3H); ¹³C NMR (101 MHz, CDCl₃) δ 172.94, 147.15, 142.95, 140.01, 131.40, 130.46, 129.30, 128.52, 128.48, 128.21, 128.07, 127.85, 127.51, 122.42, 119.49, 34.76, 31.47, 29.28, 27.21, 26.65, 25.61, 25.58, 25.54, 25.24, 22.54, 14.09; HRMS: (ESI-TOF) calculated for C₃₂H₄₁NONa [M + Na⁺] 478.3086, found 478.3080.

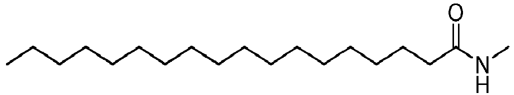

### N-methylstearamide (70)

Made according to the general procedure with methylamine, isolated as a white solid (259 mg, 91 % yield).

R_{f} = 0.24 (Pentane/EtOAc, 1/1); IR (film) υₘₐₓ 1638 cm⁻¹; ¹H NMR (400 MHz, CDCl₃) δ 5.40 (s, 1H), 2.79 (m, 3H), 2.14 (t, *J* = 7.6 Hz, 2H), 1.59 (m, 3H), 1.24 (m, 27H), 0.87 (m, 3H); ¹³C NMR (101 MHz, CDCl₃) δ 173.91, 36.86, 31.86, 29.67, 29.66, 29.65, 29.64, 29.63, 29.62, 29.61, 29.60, 29.59, 29.57, 29.46, 29.33, 29.32, 29.31, 14.07; HRMS: (ESI-TOF) calculated for C₁₉H₃₉NONa [M + Na⁺] 320.2929, found 320.2930.

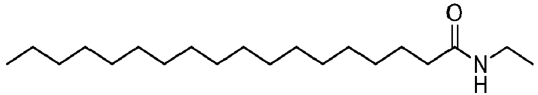

### N-ethylstearamide (71)

Made according to the general procedure with ethylamine, isolated as a white solid (263 mg, 88 % yield).

R_{f} = 0.37 (Pentane/EtOAc, 1/1); IR (film) υₘₐₓ 1640 cm⁻¹; ¹H NMR (400 MHz, CDCl₃) δ 5.36 (s 1H), 3.28 (m, 2H), 2.13 (t, *J* = 7.6 Hz, 2H), 1.60 (m, 3H), 1.25 (m, 27H), 1.12 (t, *J* = 7.6 Hz, 2H), 0.88 (m, 3H); ¹³C NMR (101 MHz, CDCl₃) δ 173.26, 36.88, 34.26, 34.10, 31.91, 26.69, 29.66, 29.64, 29.62, 29.58, 29.56, 29.46, 29.42, 29.33, 29.28, 29.23, 25.76, 22.66, 14.93, 14.10; HRMS: (ESI-TOF) calculated for C₂₀H₄₁NONa [M + Na⁺] 334.3086, found 334.3081.

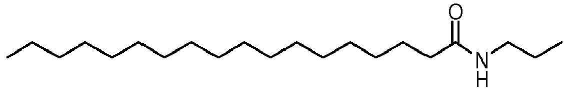

### N-propylstearamide (72)

Made according to the general procedure with *n*-propylamine, isolated as a white solid (268 mg, 86 % yield).

R_{f} = 0.48 (Pentane/EtOAc, 1/1); IR (film) υₘₐₓ 1639 cm⁻¹; ¹H NMR (400 MHz, CDCl₃) δ 5.40 (s, 1H), 3.24 (q, *J* = 6.9 Hz, 2H), 2.11 (t, *J* = 6.9 Hz, 3H), 1.60 (m, 3H), 1.50 (m, 3H), 1.25 (m, 22H), 0.89 (m, 9H);¹³C NMR (101 MHz, CDCl₃) δ 173.12, 41.18, 36.94, 31.87, 29.68, 29.67, 29.66, 29.65, 29.60, 29.59, 29.58, 29.46, 29.33, 29.29, 25.78, 22.91, 22.66, 14.10, 11.30; HRMS: (ESI-TOF) calculated for C₂₁H₄₃NONa [M + Na⁺] 348.3242, found 348.3257.

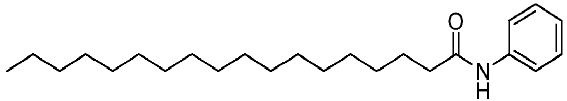

### N-phenylstearamide (73)

Made according to the general procedure with aniline, isolated as a white solid (314 mg, 91 % yield).

R_{f} = 0.55 (Pentane/EtOAc, 1/1); IR (film) υₘₐₓ 1652 cm⁻¹; ¹H NMR (400 MHz, CDCl₃) δ 7.50 (d, *J* = 7.9 Hz, 2H), 7.30 (t, app. *J* = 7.9 Hz, 2H), 7.09 (m, 2H), 2.33 (t, *J* = 7.6 Hz, 2H), 1.71 (m, 28H), 0.86 (m, 3H); ¹³C NMR (101 MHz, CDCl₃) δ 171.85, 137.85, 128.94, 124.17, 119.64, 37.89, 31.93, 29.69, 29.67, 29.66, 29.64, 29.63, 29.62, 29.60, 29.58, 29.45, 29.35, 29.33, 29.24, 25.61, 22.65, 14.10; HRMS: (ESI-TOF) calculated for C₂₄H₄₁NONa [M + Na⁺] 382.3086, found 382.3095.

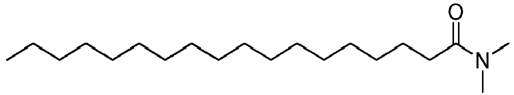

### N,N-dimethylstearamide (74)

Made according to the general procedure with dimethylamine, isolated as a white solid (260 mg, 87 % yield).

R_{f} = 0.24 (Pentane/EtOAc, 1/1); IR (film) υₘₐₓ 1638 cm⁻¹; NMR (400 MHz, CDCl₃) δ 2.97 (s, 3H), 2.91 (s, 3H), 2.27 (t, *J* = 7.1 Hz, 2H), 1.59 (m, 2H), 1.28 (m, 28H), 0.85 (m, 3H); ¹³C NMR (101 MHz, CDCl₃) δ 173.26, 37.30, 35.34, 33.40, 31.84, 29.69, 29.66, 29.65, 29.64, 29.63, 29.62, 29.61, 29.59, 29.56, 29.54, 29.50, 25.17, 22.66, 14.04; HRMS: (ESI-TOF) calculated for C₂₀H₄₁NONa [M + Na⁺] 334.3086, found 334.3090.

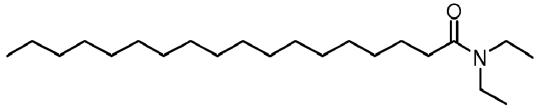

### N,N-diethylstearamide (75)

Made according to the general procedure with diethylamine, isolated as a white solid (234 mg, 72 % yield).

R_{f} = 0.39 (Pentane/EtOAc, 1/1); IR (film) υₘₐₓ 1642 cm⁻¹; ¹H NMR (400 MHz, CDCl₃) δ 3.35 (q, *J* = 7.1 Hz, 2H), 3.28 (q, *J* = 7.1 Hz, 2H), 2.26 (t, *J* = 7.5 Hz, 2H), 1.61 (m, 2H), 1.26 (m, 28H), 1.15 (t, app. *J* = 7.1 Hz, 3H), 1.09 (t, app. *J* = 7.1 Hz, 3H), 0.86 (m,3H); ¹³C NMR (101 MHz, CDCl₃) δ 172.16, 41.89, 39.93, 33.14, 31.92, 29.67, 29.66, 29.64, 29.63, 29.62, 29.61, 29.53, 29.51, 29.47, 29.32, 25.51, 22.69, 14.37, 14.05, 13.11; HRMS: (ESI-TOF) calculated for C₂₂H₄₅NONa [M + Na⁺] 362.3399, found 362.3406.

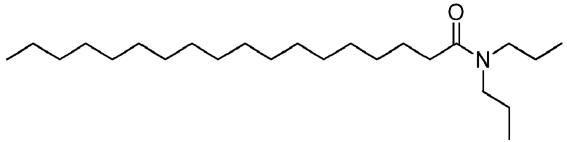

### N,N-dipropylstearamide (76)

Made according to the general procedure with *n*,*n*-dipropylamine, isolated as a white solid (278 mg, 79 % yield).

R_{f} = 0.51 (Pentane/EtOAc, 1/1); IR (film) υₘₐₓ 1641 cm⁻¹; ¹H NMR (400 MHz, CDCl₃) δ 3.25 (m, 2H), 3.15 (m, 2H), 2.26 (t, *J* = 7.7 Hz, 2H), 1.57 (m, 6H), 1.25 (m, 29H), 0.88 (m, 8H); ¹³C NMR (101 MHz, CDCl₃) δ 172.56, 49.61, 47.41, 33.21, 31.86, 29.68, 29.66, 29.64, 29.63, 29.62, 29.60, 29.50, 29.48, 29.33, 25.55, 22.66, 22.31, 20.95, 14.10, 11.39, 11.23; HRMS: (ESI-TOF) calculated for C₂₂H₄₉NONa [M + Na⁺] 390.3712, found 390.3708.

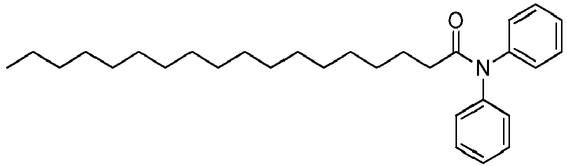

### N,N-diphenylstearamide (77)

Made according to the general procedure with diphenylamine, isolated as a white solid (250 mg, 59 % yield).

R_{f} = 0.53 (Pentane/EtOAc, 25/1); IR (film) υₘₐₓ 1651 cm⁻¹ ; ¹H NMR (400 MHz, CDCl₃) δ 7.26 (t, *J* = 7.9 Hz, 4H), 7.07 (d, app. *J* = 7.4 Hz, 2H), 6.92 (t, app., *J* = 7.4 Hz, 2H), 2.25 (t, *J* = 6.5 Hz, 2H), 1,66 (m, 2H), 1.25 (m, 28H), 0.88 (m, 3H); ¹³C NMR (101 MHz, CDCl₃) δ 173.37, 143.07, 129.24, 121.03, 117.79, 35.27, 31.88, 29.74, 29.72, 29.69, 29.67, 29.66, 29.64, 29.63, 29.45, 29.36, 29.35, 29.33, 29.23, 25.58, 22.68, 14.13; HRMS: (ESI-TOF) calculated for C₃₀H₄₅NONa [M + Na⁺] 458.3399, found 458.3408.

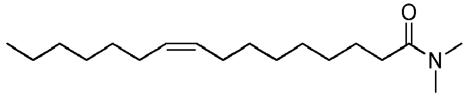

### N,N-dimethylpalmitoleamide (89)

Made according to the general procedure with dimethylamine, isolated as a clear oil (470 mg, 88 % yield).

R_{f} = 0.29 (Pentane/EtOAc, 1/1); IR (film) υₘₐₓ 1639, 1465 cm^{-1 1}H NMR (400 MHz, CDCl₃) δ 5.33 (m, 2H), 2.98 (s, 3H), 2.90 (s, 3H), 2.26 (t, *J* =7.2 Hz, 2H), 1.97 (m, 4H), 1.62 (m, 2H), 1.28 (m, 16H), 0.65 (m, 3H); ¹³C NMR (101 MHz, CDCl₃) δ 173.52, 129.85, 129.76, 37.82, 35.74, 33.56, 31.89, 29.92, 29.70, 29.67, 29.24, 29.23, 29.07, 28.97, 27.14, 25.58, 14.07; HRMS: (ESI-TOF) calculated for C₁₈H₃₅NONa [M + Na⁺] 304.2616, found 304.2617.

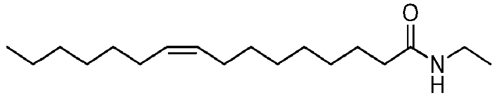

### N-ethylpalmitoleamide (90)

Made according to the general procedure with ethylamine, isolated as a clear oil (319 mg, 71 % yield).

R_{f} = 0.33 (Pentane/EtOAc, 1/1); IR (film) υₘₐₓ 1641, 1462 cm⁻¹; ¹H NMR (400 MHz, CDCl₃) δ 5.32 (m, 3H), 3.27 (m, 2H), 2.13 (t, app. *J* = 7.4 Hz, 2H), 1.99 (m, 4H), 1.61 (m, 2H), 1.28 (m, 16H), 1.12 (t, *J* = 7.4 Hz, 3H), 0.87 (m, 3H); ¹³C NMR (101 MHz, CDCl₃) δ 172.95, 129.95, 129.72, 36.81, 34.26, 31.72, 29.70, 29.67, 29.25, 29.23, 29.10, 28.95, 27.18, 27.13, 25.75, 22.68, 14.89, 14.14; HRMS: (ESI-TOF) calculated for C₁₈H₃₅NONa [M + Na⁺] 304.2616, found 304.2622.

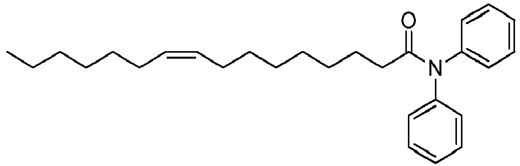

### N,N-diphenylpalmitoleamide (91)

Made according to the general procedure with diphenylamine, isolated as a clear oil (470 mg, 61 % yield).

R_{f} = 0.59 (Pentane/EtOAc, 25/1); IR (film) υₘₐₓ 1650, 1467 cm⁻¹; ¹H NMR (400 MHz, CDCl₃) δ 7.26 (m, 4H), 7.06 (m, 4H), 6.91 (m, 2H), 2.24 (t, *J* = 7.4 Hz, 2H), 1.98 (m, 4H), 1.64 (m, 2H), 1.26 (m, 18H), 0.62 (m, 3H); ¹³C NMR (101 MHz, CDCl₃) δ 173.65, 143.26, 129.93, 129.76, 129.16, 121.25, 117.96, 35.29, 31.77, 29.71, 29.68, 29.23, 29.21, 29.19, 29.09, 28.95, 28.68, 27.80, 27.19, 27.15, 25.54, 14.08; HRMS: (ESI-TOF) calculated for C₂₈H₃₉NONa [M + Na⁺] 428.2929, found 428.2948.

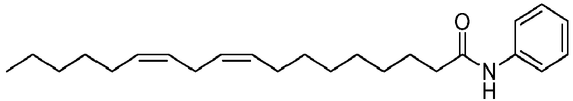

### N-phenyllinoleamide (53)

Made according to the general procedure with aniline, isolated as a clear oil (760 mg, 75 % yield).

R_{f} = 0.50 (Pentane/EtOAc, 1/1); IR (film) υₘₐₓ 1651, 1466, 1429 cm⁻¹ ; ¹H NMR (400 MHz, CDCl₃) δ 7.49 (d, *J* = 7.9 Hz, 2H), 7.30 (t, *J* = 7.9 Hz, 2H), 7.08 (m, 2H), 5.34 (m, 4H), 2.76 (t, *J* = 7.1 Hz, 2H), 2.33 (t, *J* = 7.6 Hz, 2H), 2.03 (m, 4H), 1.71 (m, 2H), 1.31 (m, 14H), .087 (m, 3H); ¹³C NMR (101 MHz, CDCl₃) δ 171.07, 137.90, 130.17, 130.00, 128.95, 128.02, 127.86, 37.89, 31.47, 29.57, 29.56, 29.31, 29.25, 29.21, 29.20, 29.10, 27.17, 27.16, 25.60, 25.57, 22.57, 14.02; HRMS: (ESI-TOF) calculated for C₂₄H₃₈NO [M + H⁺] 356.2953, found 356.2962.

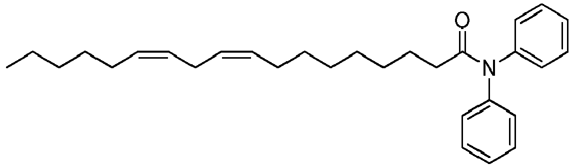

### N,N-diphenyllinoleamide (54)

Made according to the general procedure with diphenylamine, isolated as a clear oil (600 mg, 49 % yield).

R_{f} = 0.51 (Pentane/EtOAc, 25/1); IR (film) υₘₐₓ 1652, 1468, 1432 cm⁻¹ ; ¹H NMR (400 MHz, CDCl₃) δ 7.38 (m, 4H), 7.24 (m, 4H), 7.10 (m, 2H), 5.30 (m, 4H), 2.74 (t, *J* = 6.7 Hz, 2H), 2.23 (t, *J* = 7.2 Hz, 2H), 1.63 (m, 4H), 1.28 (m, 16 H), 0.87 (m, 3H); ¹³C NMR (101 MHz, CDCl₃) δ 172.26, 139.78, 130.04, 129.30, 129.07, 128.99, 128.43, 126.99, 126.22, 35.25, 31.49, 29.68, 29.58, 29.32, 29.22, 29.18, 29.09, 27.14, 25.59, 25.48, 22.53, 14.03; HRMS: (ESI-TOF) calculated for C₃₀H₄₂NO [M + H⁺] 432.3266, found 432.3274.

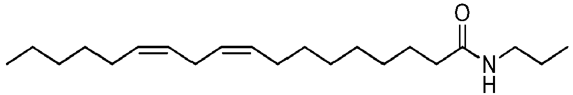

### N-propyllinoleamide (58)

Made according to the general procedure with *n*-propylamine, isolated as a clear oil (586 mg, 64 % yield).

R_{f} = 0.34 (Pentane/EtOAc, 1/1); IR (film) υₘₐₓ 1644, 1555, 1460 cm⁻¹ ; ¹H NMR (400 MHz, CDCl₃) δ 5.32 (m, 4H), 3.20 (q, *J* = 7.2 Hz, 2H), 2.75 (t, *J* = 6.5 Hz, 2H), 2.13 (t, *J* = 7.5 Hz, 2H), 2.02 (m, 4H), 1.61 (m, 2H), 1.49 (m, 2H), 1.29 (m, 16H), 0.89 (m, 5H); ¹³C NMR (101 MHz, CDCl₃) δ 172.95, 130.22, 128.02, 127.86, 41.13, 36.87, 31.49, 29.58, 29.51, 29.31, 29.25, 29.22, 29.11, 27.15, 25.76, 25.62, 22.99, 22.55, 14.09, 11.38; HRMS: (ESI-TOF) calculated for C₂₁H₃₉NONa [M + Na⁺] 344.2929, found 344.2928.

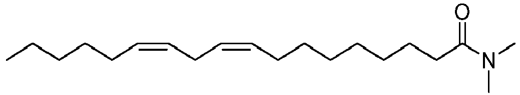

### N,N-dimethyllinoleamide (100)

Made according to the general procedure with dimethylamine, isolated as a clear oil (250 mg, 77 % yield).

R_{f} = 0.26 (Pentane/EtOAc, 1/1); IR (film) υₘₐₓ 1643, 1554, 1461 cm^{-1 1}H NMR (400 MHz, CDCl₃) δ 5.32 (m, 4H), 2.98 (s, 3H), 2.92 (s, 3H), 2.75 (t, *J* = 6.8 Hz, 2H), 2.29 (t, *J* = 7.8 Hz, 2H), 2.03 (q, *J* = 6.8 Hz, 2H), 1.61 (m, 2H), 1.30 (m, 16 H), 0.86 (m, 3H); ¹³C NMR (101 MHz, CDCl₃) δ 172.95, 130.17, 130.06, 127.96, 127.89, 37.26, 35.30, 33.39, 31.50, 29.60, 29.46, 29.34, 29.32, 29.15, 27.15, 25.62, 25.20, 22.62, 14.01; HRMS: (ESI-TOF) calculated for C₂₀H₃₇NONa [M + Na⁺] 330.2773, found 330.2772.

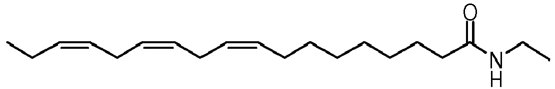

### N-ethyllinolenamide (57)

Made according to the general procedure with ethylamine, isolated as a white solid (596 mg, 68 % yield).

R_{f} = 0.29 (Pentane/EtOAc, 1/1); IR (film) υₘₐₓ 1641, 1462 cm⁻¹ ; ¹H NMR (400 MHz, CDCl₃) δ 5.34 (m, 6H), 3.20 (m, 3H), 2.79 (m, 4H), 2.12 (m, 2H), 2.04 (m, 4H), 1.60 (m, 2H), 1.30 (m, 9H), 1.11 (t, *J =* 6.4 Hz, 3H), 0.96 (t, *J* = 7.5 Hz, 2H); ¹³C NMR (101 MHz, CDCl₃) δ 172.56, 131.92, 130.23, 128.24, 128.22, 127.66, 127.08, 36.96, 34.24, 29.57, 29.55, 29.25, 29.23, 29.09, 27.18, 25.76, 25.59, 25.49, 20.57, 14.96; HRMS: (ESI-TOF) calculated for C₂₀H₃₅NONa [M + Na⁺] 328.2616, found 328.2628.

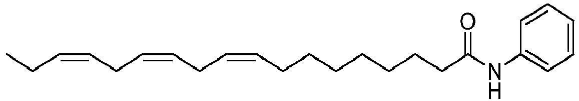

### N-phenyllinolenamide (59)

Made according to the general procedure with aniline, isolated as a clear oil (730 mg, 72 % yield).

R_{f} = 0.77 (Pentane/EtOAc, 1/1); IR (film) υₘₐₓ 1650, 1460 cm⁻¹ ; ¹H NMR (400 MHz, CDCl₃) δ 7.49 (d, *J* = 7.9 Hz, 1H), 7.29 (t, app. *J* = 7.2 Hz, 2H), 7.08 (t, app. *J* = 7.2 Hz, 2H), 5.34 (m, 6H), 2.79 (t, *J* = 7.0 Hz, 2H), 2.33 (t, *J* = 7.6 Hz, 2H), 2.05 (m, 4H), 1.71 (m, 4H), 1.31 (m, 11H), 0.96 (t, *J* = 7.0 Hz, 3H); ¹³C NMR (101 MHz, CDCl₃) δ 171.01, 132.87, 131.96, 130.23, 129.98, 129.25, 128.96, 127.70, 127.07, 124.14, 119.73, 37.85, 31.50, 29.55, 29.25, 29.21, 29.10, 27.18, 25.60, 22.56, 20.53, 14.29; HRMS: (ESI-TOF) calculated for C₂₄H₃₅NONa [M + Na⁺] 376.2616, found 376.2605.

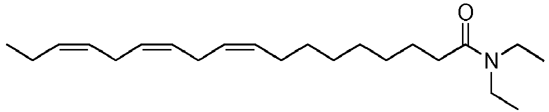

### N,N-diethyllinolenamide (60)

Made according to the general procedure with diethylamine, isolated as a clear oil (957 mg, 71 % yield).

R_{f} =0.29 (Pentane/EtOAc, 1/1); IR (film) υₘₐₓ 1640, 1465 cm^{-1 1}H NMR (400 MHz, CDCl₃) δ 5.36 (m, 6H), 3.35 (q, *J* = 7.1 Hz, 3H), 3.28 (q, *J* = 7.1 Hz, 3H), 2.78 (m, 4H), 2.26 (t, *J* = 7.3 Hz, 2H), 2.05 (m, 4H), 1.61 (m, 3H), 1.31 (m, 5H), 1.14 (t, *J* = 7.1 Hz, 3H), 1.08 (t, *J* = 7.1 Hz, 3H), 0.93 (m, 3H); ¹³C NMR (101 MHz, CDCl₃) δ 172.16, 131.90, 130.29, 128.22, 127.96, 127.65, 127.09, 41.87, 39.99, 33.15, 31.49, 29.59, 29.48, 29.37, 29.15, 27.19, 25.59, 25.46, 14.39, 13.08; HRMS: (ESI-TOF) calculated for C₂₂H₄₀NO [M + H⁺] 334.3110, found 334.3108.

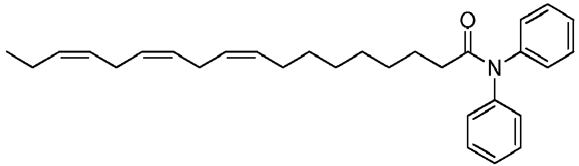

### N,N-diphenyllinolenamide (62)

Made according to the general procedure with diphenylamine, isolated as a clear oil (665 mg, 54 % yield).

R_{f} = 0.61 (Pentane/EtOAc, 25/1); IR (film) υₘₐₓ 1651, 1464 cm⁻¹; ¹H NMR (400 MHz, CDCl₃) δ 7.27 (m, 4H), 7.07 (m, 4H), 6.90 (m, 2H), 5.33 (m, 6H), 2.81 (t, *J* = 7.1 Hz, 2H), 2.32 (t, *J* = 7.6 Hz, 2H), 2.05 (m, 4H), 1.68 (m, 4H), 1.30 (m, 8H), 0.94 (m, 3H); ¹³C NMR (101 MHz, CDCl₃) δ 171.21, 133.96, 132.87, 131.74, 130.25, 129.68, 129.64, 129.54, 127.92, 124.56, 119.78, 37.86, 32.60, 29.78, 29.56, 29.42, 29.38, 29.15, 27.18, 25.42, 20.68, 14.34; HRMS: (ESI-TOF) calculated for C₃₀H₃₉NONa [M + Na⁺] 452.2929, found 452.2937.

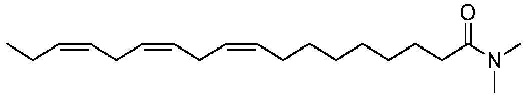

### N,N-dimethyllinolenamide (99)

Made according to the general procedure with dimethylamine, isolated as a clear oil (441 mg, 76 % yield).

R_{f} = 0.27 (Pentane/EtOAc, 1/1); IR (film) υₘₐₓ 1639, 1462 cm⁻¹; ¹H NMR (400 MHz, CDCl₃) δ 5.35 (m, 6H), 2.99 (s, 3H), 2.92 (s, 3H), 2.77 (m, 4H), 2.28 (t, *J* = 7.9 Hz, 3H), 2.04 (m, 4H), 1.61 (m, 3H), 1.32 (m, 5H), 0.96, (t, *J* = 7.5 Hz, 2H), 0.87 (m, 2H); ¹³C NMR (101 MHz, CDCl₃) δ 172.26, 131.92, 130.26, 128.25, 127.96, 127.68, 127.11, 37.31, 35.32, 33.38, 31.44, 29.57, 29.47, 29.32, 29.17, 27.11, 25.11, 20.50, 14.01; HRMS: (ESI-TOF) calculated for C₂₀H₃₅NONa [M + Na⁺] 328.2616, found 328.2619.

### In vivo LPS Cell Model

An *in vivo* study (LPS Shock Model) was performed on BALB/c female mice aged 17-19 weeks. Mice were administered 10 mg/kg oleamide via IP injection at the start of the study and one hour later. Two hours after the final oleamide IP injection, mice were administered 3µg lipopolysaccharide (LPS) via IV injection. Six hours after Lipopolysaccharide (LPS) IV injection, the mice were culled and whole blood was collected by cardiac puncture in serum micro tubes (Sarstedt, Germany). The whole blood was stored at 2-4°C overnight to allow for clotting and micro tubes were subsequently centrifuged at 1200 RPM for 5 minutes. The serum was removed and added into sterile Eppendorf's. Specific cytokine levels (IL-12p40, INF-gamma and IL-1β) were measured by ELISA.

Oleamide was dissolved in 98% Ethanol (Sigma, USA) at a concentration of 10 mg/ml. Subsequently, this solution was diluted in sterile PBS (Life Technologies, Ireland) at a working concentration of 1 mg/ml. This solution was prepared freshly before use.

LPS (from *E.coli*, Serotype R515) (Enzo Life Sciences, UK) was prepared at a concentration of 0.03 mg/ml in sterile PBS. This solution was prepared freshly before use.

The concentration of cytokines in cell supernatants was determined using ELISA Duoset kits from R&D Systems in accordance with the manufacturers' instructions. Briefly, 100 µl/well of capture antibody (diluted to the appropriate concentration in PBS) was added to an ELISA plate (Nunc) and incubated overnight at room temperature. Plates were then washed three times with Wash Buffer (PBS + 0.05% Tween-20) and blocked by adding 300 µl of Blocking buffer (1% (w/v) BSA/PBS) for a minimum of 1h at room temperature. The washing step was repeated and 100 µl of samples and standards (diluted to appropriate concentrations) were added per well, in triplicate. Plates were then incubated at 2-4°C overnight. The next day, plates were washed again and 100 µl of the biotinylated detection antibody was added to each well and incubated for 2 hours at room temperature. 100 µl of Streptavidin- horseradish-peroxidase (R&D Systems) was then added to each well, following another wash step. Plates were incubated for 20min at room temperature. After the last washing step, 100 µl of tetramethylbenzidine (BD Biosciences) was added to each well and plates were left in the dark for 20 minutes or until a blue colour developed. Colour development was stopped by adding 50 µl of 1M sulphuric acid (Sigma-Aldrich) to the wells. The optical density was determined at 450nm, using a VersaMax™ microplate reader (Molecular Devices). For each set of samples, a standard curve was generated and the values of unknown samples were calculated from the standard curve.

### In vitro: Screening Oleamide and Analogues

Bone marrow derived dendritic cells (BMDCs) were cultured as follows:
(Day 1) Bone marrow cells were harvested from the femur and tibia of Balb/c female mice by flushing the bone with RPMI 1640 complete media [supplemented with 10% foetal calf serum and 2% Penicilin-Steptomycin (Life Technologies, Ireland)] using a 27 gauge needle attached to a 10ml syringe. The bone marrow cells were collected into a 50-ml Falcon tube and centrifuged at 1200 RPM for 5 minutes. In order to derive the bone marrow cells into dendritic cells, the pellet was resuspended into appropriate volume of complete media which was supplemented with 5 ng/ml of recombinant GM-CSF (Sigma-Aldrich, USA). Generally, one bone was used per sterile petri plate for culturing. Cells were grown in an incubator at 37 °C, 95% humidity and 5% carbon dioxide until day 4.
(Day 4) Media was changed in each petri plate by removing 6-7 mls of old media using a Pasteur pipette and adding 10 ml of fresh complete media containing recombinant GM-CSF. Cells were grown in an incubator at 37 °C, 95% humidity and 5% Carbon dioxide until day 8.
(Day 8) The BMDCs were collected into a 50-ml Falcon tube by using a cell scraper and Pasteur pipette for collection and transferring cells. BMDCs were centrifuged at 1200 RPM for 5 minutes and resuspended in 10 ml of complete media without GM-CSF. The cells were counted using the typan blue exclusion test for viability (Sigma-Aldrich, USA) and plated at 1x10⁶ cells per ml on 24 well plates (Nunc) and left to settled in the incubator at 37°C, 95% humidity and 5% carbon dioxide.

The BMDCs were conditioned with 25 uM oleamide and analogues one hour prior to stimulation with 100 ng/ml LPS (*E.Coli* serotype R515). After 24 hours, the supernatants were removed and analysed for cytokine levels of IL-12p40, IL-12p70, IL-23 and TNF-alpha using specific immunoassays (as described above).

### In vitro Cell Viability

Bone Marrow Dendritic cells (BMDCs) were cultured *in vitro* and 100ul of cells were plated on a sterile 96-well plate (Nunc) at a concentration of 1 x 10⁶ cells per ml (as previously described). CellTiter 96® AQueous One Solution Cell Proliferation Assay (MTS) (Promega, USA) was performed in order to determine the viability of the BMDCs when they were associated with oleamide and the analogues. This was carried out by conditioning the BMDCs with 25uM of oleamide and 25 uM of each analogue for 24 hours. The 96-well plate was incubated at 37°C in a humidified, 5% CO₂ atmosphere. To this end, 20 ul of the CellTiter96® Aqueous One Solution was added to each well of the 96-well plate containing samples. The absorbance was recorded at 490nm using a 96-well spectrophotometer at time points of 1, 2 and 3 hours (after each reading the plate was placed back in the incubator). The optical density was determined at 490nm, using a VersaMax™ microplate reader (Molecular Devices). Cells alone were represented as 100% viable and each set of samples was referenced to this control.

**Table 1. An overview of Oleamide and its analogues and the effects they have on modulating cytokine secretion (in vitro)**

| **Group** | **Reference Number** | **Name/Structure/Molecular Weight (g/mol)** | | **+LPS** | | | | **Category*** |
|---|---|---|---|---|---|---|---|---|
| | | | | **IL-12p40** | **IL-12p70** | **IL-23** | **TNF-Alpha** | |
| Oleamide | Ole | 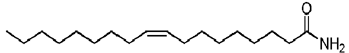 | | 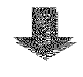 | 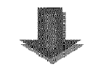 | 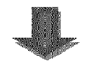 | 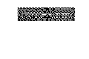 | 1,5 |
| | | Oleamide mw: 281.47660 | | | | | | |
| | 37 | 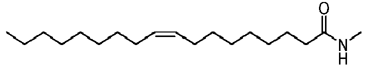 | | 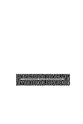 | 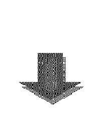 | 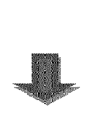 | 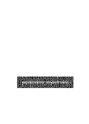 | 4 |
| | | MC 6-37 *N*-methyloleamide mw: 295.50318 | | | | | | |
| | 38 | 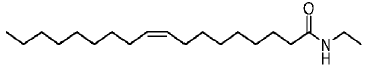 | | 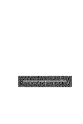 | 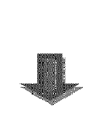 | 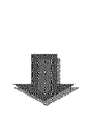 | 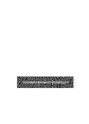 | 4 |
| | | MC 6-38 *N*-ethyloleamide mw: 309.52976 | | | | | | |
| | 39 | 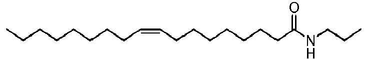 | | 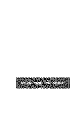 | 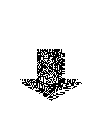 | 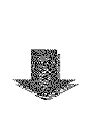 | 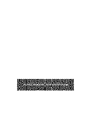 | 4 |
| | | MC 6-39 *N*-propyloleamide mw: 323.55634 | | | | | | |
| | 40 | 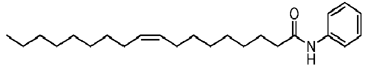 | | 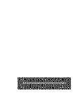 | 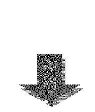 | 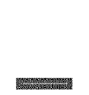 | 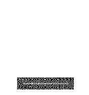 | 4 |
| | | MC 6-40 *N*-phenyloleamide mw: 357.57256 | | | | | | |
| Oleamide | 41 | 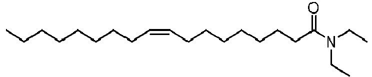 | | 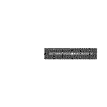 | 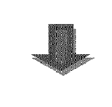 | 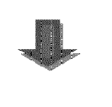 | 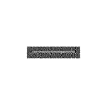 | 4 |
| | | MC 6-41 *N,N*-diethyloleamide mw: 337.58292 | | | | | | |
| | 43 | 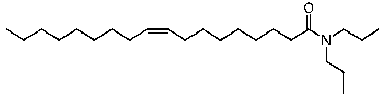 | | 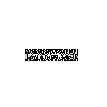 | 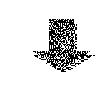 | 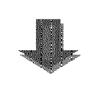 | 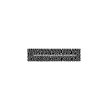 | 4 |
| | | MC6-43 *N,N*-dipropyloleamide mw: 365.63608 | | | | | | |
| | 44 | 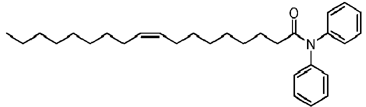 | | 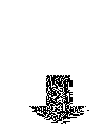 | 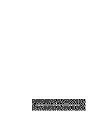 | 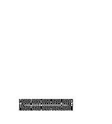 | 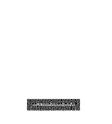 | 4 |
| | | MC 6-44 *N,N*-diphenyloleamide mw: 433.66852 | | | | | | |
| | 69 | 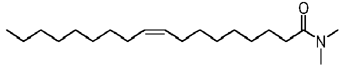 | N,N-dimethyloleamide MC 6-69 mw: 309.529 g/mol | 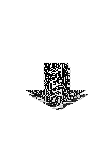 | 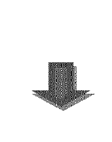 | 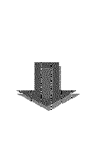 | 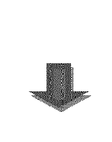 | 3 |
| | | | mass in vial: 107.9 mg | | | | | |
| Oleamide | 104 | 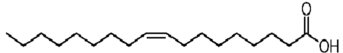 | oleic acid MC 6-104 mw: 282.461 g/mol | 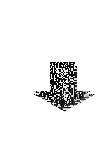 | 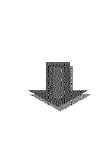 | 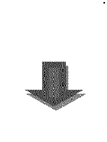 | 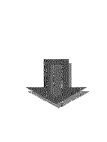 | 3 |
| | | | mass in vial: 26.8 mg | | | | | |
| Palmitamide | 78 | 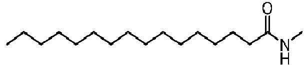 | N-methylpalmitamide MC 6-78 mw: 269.465 g/mol | 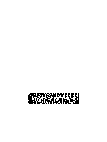 | 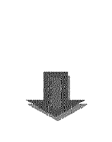 | 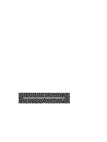 | 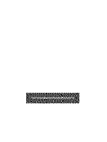 | 4 |
| | | | mass in vial: 84.6 mg | | | | | |
| | 79 | 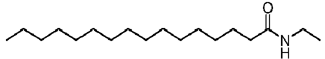 | N-ethylpalmitamide MC 6-79 mw: 283.492 g/mol | 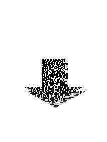 | 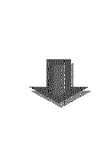 | 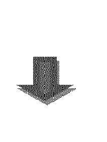 | 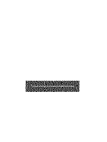 | 1,5 |
| | | | mass in vial: 50.5 mg | | | | | |
| | 80 | 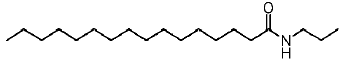 | N-propylpalmitamide MC 6-80 mw: 297.519 g/mol | 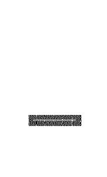 | 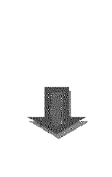 | 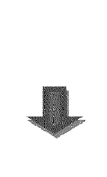 | 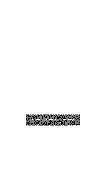 | 4 |
| | | | mass in vial: 63.5 mg | | | | | |
| Palmitamide | 81 | 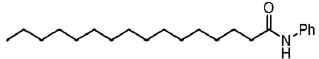 | N-methylpalmitamide MC 6-81 mw: 331.535 g/mol | 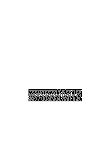 | 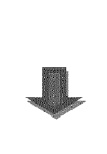 | 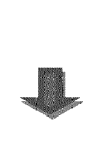 | 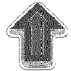 | 3 |
| | | | mass in vial: 67.9 mg | | | | | |
| | 82 | 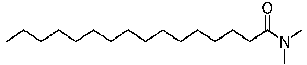 | N,N-dimethylpalmitamide MC 6-82 mw: 283.492 g/mol | 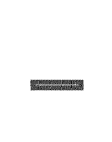 | 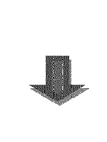 | 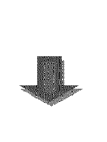 | 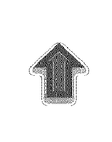 | 4 |
| | | | mass in vial: 104.1 mg | | | | | |
| | 83 | 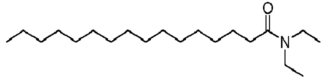 | N,N-diethylpalmitamide MC 6-83 mw: 311.545 g/mol | 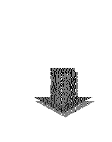 | 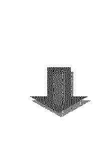 | 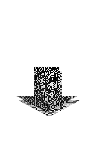 | 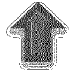 | 4 |
| | | | mass in vial: 96.1 mg | | | | | |
| | 84 | 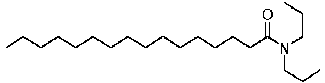 | N,N-dipropylylpalmitamide MC 6-84 mw: 339.598 g/mol | 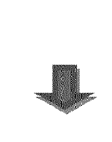 | 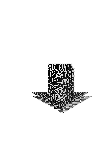 | 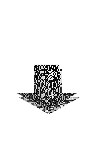 | 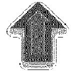 | 4 |
| | | | mass in vial: 36.6 mg | | | | | |
| Palmitamide | 85 | 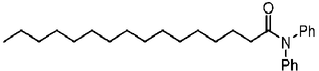 | N,N-diphenylpalmitamide MC 6-85 mw: 407.631 g/mol | 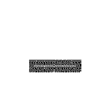 | 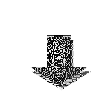 | 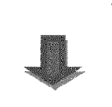 | 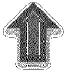 | 4 |
| | | | mass in vial: 180.8 mg | | | | | |
| | 65 | 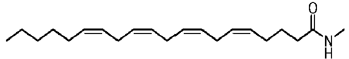 | N-methylarachidonamide MC 6-65 mw: 317.508 g/mol | 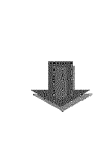 | 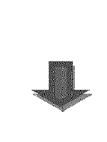 | 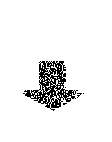 | 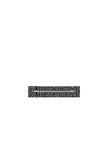 | 1,5 |
| | | | mass in vial: 20.9 mg | | | | | |
| Arachidonamide | 66 | 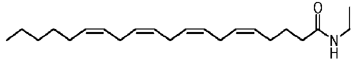 | N-ethylarachidonamide MC 6-66 mw: 331.535 g/mol | 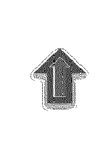 | 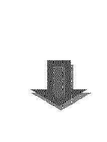 | 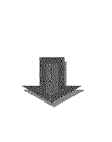 | 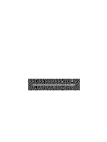 | 4 |
| | | | mass in vial: 19.4 m | | | | | |
| | 67 | 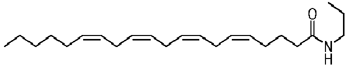 | N-propylarachidonamide MC 6-67 mw 345.561 g/mol | 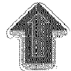 | 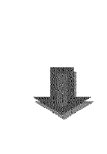 | 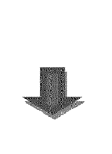 | 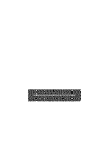 | 4 |
| | | | mass in vial: 26.4 mg | | | | | |
| Arachidonamide | 68 | 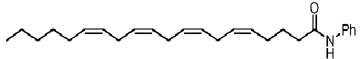 | N-phenylarachidonamide MC 6-68 mw: 379.578 g/mol | 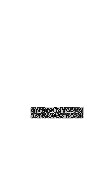 | 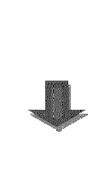 | 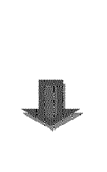 | 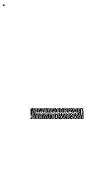 | 4 |
| | | | mass in vial: 71.2 mg | | | | | |
| | 92 | 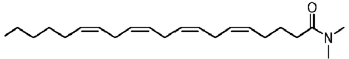 | N,N-dimethylarachidonamide MC 6-92 mw: 331.535 g/mol | 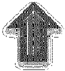 | 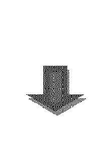 | 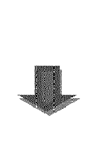 | 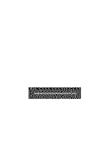 | 4 |
| | | | mass in vial: 34.9 mg | | | | | |
| | 93 | 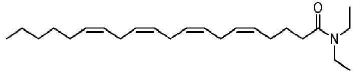 | N,N-diethylarachidonamide MC 6-93 mw: 359.588 g/mol | 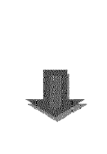 | 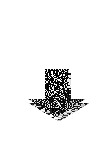 | 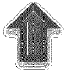 | 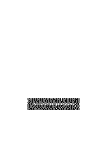 | 4 |
| | | | mass in vial: 37.3 mg | | | | | |
| | 94 | 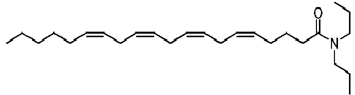 | N,N-dipropylarachidonamide MC 6-94 mw: 387.641 g/mol | 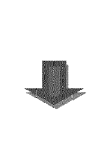 | 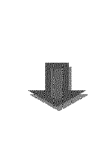 | 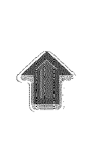 | 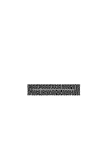 | 4 |
| | | | mass in vial: 25.0 mg | | | | | |
| Arachidonamide | 95 | 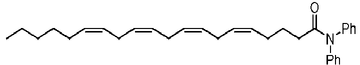 | N,N-diphenylarachidonamide MC 6-95 mw: 455.674 g/mol | 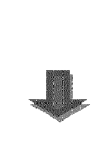 | 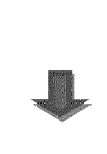 | 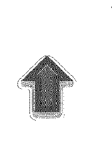 | 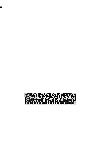 | 4 |
| | | | mass in vial: 36.0 mg | | | | | |
| Stearamide | 70 | 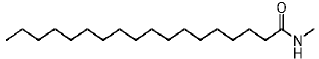 | N-methylstearamide MC 6-70 mw: 297.519 g/mol | 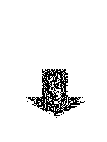 | 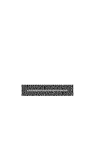 | 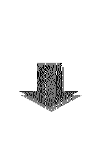 | 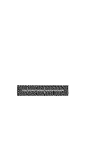 | 4 |
| | | | mass in vial: 66.4 mg | | | | | |
| | 71 | 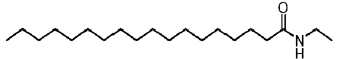 | N-ethylstearamide MC 6-71 mw: 311.545 g/mol | 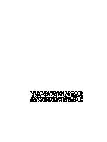 | 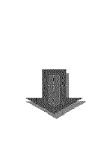 | 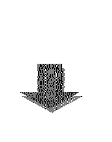 | 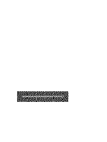 | 4 |
| | | | mass in vial: 49.3 mg | | | | | |
| | 72 | 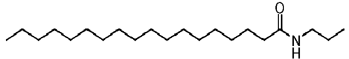 | N-propylstearamide MC 6-72 mw: 325.572 g/mol | 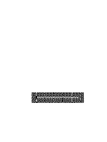 | 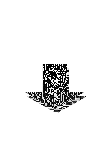 | 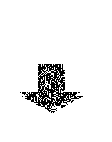 | 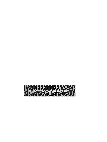 | 4 |
| | | | mass in vial: 58.0 mg | | | | | |
| Stearamide | 73 | 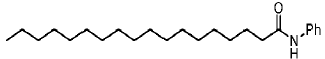 | N-phenylstearamide MC 6-73 mw: 359.588 g/mol | 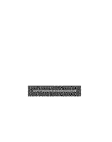 | 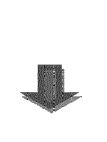 | 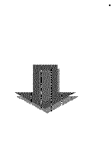 | 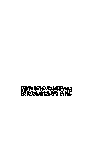 | 4 |
| | | | mass in vial: 57.5 mg | | | | | |
| | 74 | 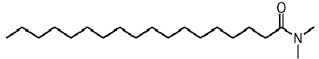 | N,N-dimethylstearamide MC 6-74 mw: 311.545 g/mol | 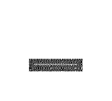 | 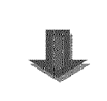 | 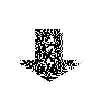 | 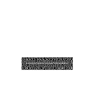 | 4 |
| | | | mass in vial: 81.3 mg | | | | | |
| | 75 | 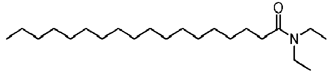 | N,N-dimethylstearamide MC 6-75 mw: 339.598 g/mol | 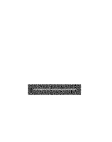 | 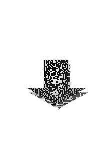 | 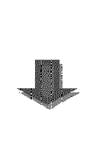 | 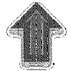 | 4 |
| | | | mass in vial: 81.3 mg | | | | | |
| | 76 | 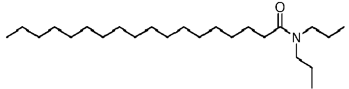 | N,N-dipropylylstearamide MC 6-76 mw: 367.651 g/mol | 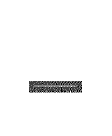 | 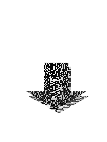 | 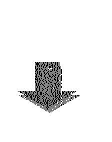 | 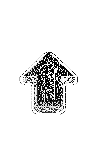 | 4 |
| | | | mass in vial: 91.1 mg | | | | | |
| Stearamide | 77 | 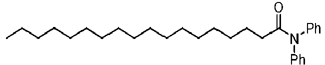 | N,N-dimethylstearamide MC 6-77 mw: 435.684 g/mol | 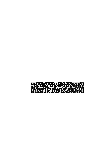 | 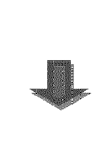 | 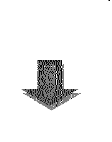 | 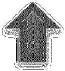 | 4 |
| | | | mass in vial: 108.8 mg | | | | | |
| Unknown Group 1 | 101 | 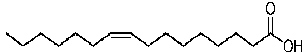 | palmitoleic acid MC 6-101 mw: 254.408 g/mol | 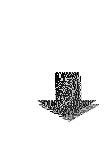 | 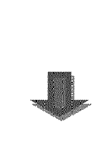 | 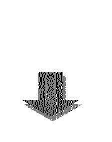 | 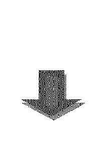 | 2,5 |
| | | | mass in vial: 39.3 mg | | | | | |
| | 89 | 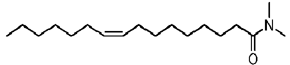 | N,N-dipmethylpalmitoleamide MC 6-89 mw: 281.476 g/mol | 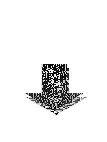 | 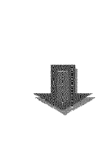 | 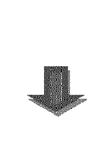 | 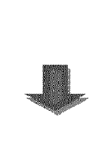 | 2,5 |
| | | | mass in vial: 25.5 mg | | | | | |
| | 90 | 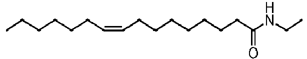 | N,N-diphenylpalmitoleamide MC 6-90 mw: 281.476 g/mol | 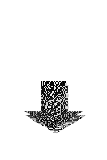 | 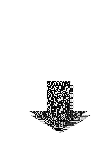 | 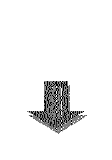 | 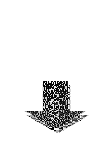 | 2,5 |
| | | | mass in vial: 24.8 mg | | | | | |
| Unknown Group 1 | 91 | 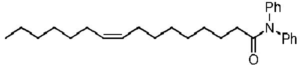 | N,N-diphenylpalmitoleamide MC 6-91 mw: 405.615 g/mol | 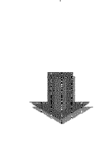 | 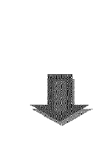 | 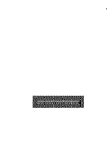 | 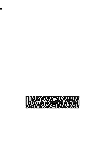 | 4 |
| | | | mass in vial: 127.2 mg | | | | | |
| Unknown Group 2 | 102 | 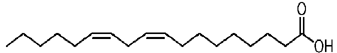 | linoleic acid MC 6-102 mw: 280.445 g/mol | 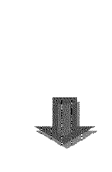 | 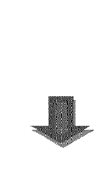 | 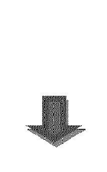 | 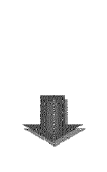 | 2,5 |
| | | | mass in vial: 157.0 mg | | | | | |
| | 53 | 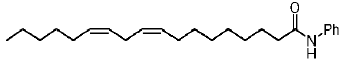 | N-phenyllinoleamide MC 6-53 mw: 355.556 g/mol | 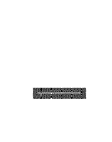 | 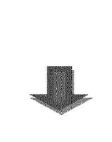 | 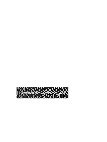 | 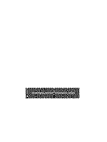 | 4 |
| | | | mass in vial: 30.7 mg | | | | | |
| | 54 | 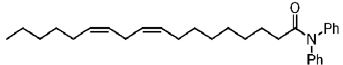 | N,N-diphenyllinoleamide MC 6-54 mw: 431.652 g/mol | 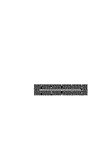 | 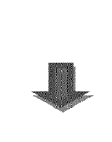 | 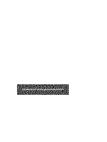 | 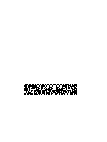 | 4 |
| | | | mass in vial: 6.5 mg | | | | | |
| Unknown Group 2 | 58 | 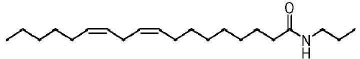 | N-propyllinoleamide MC 6-58 mw: 321.540 g/mol | 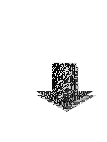 | 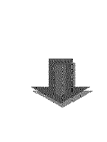 | 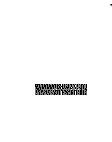 | 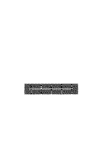 | 4 |
| | | | mass in vial 1.8 mg | | | | | |
| | 100 | 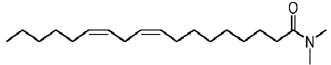 | N,N-dimethyllinoleamide MC 6-100 mw: 307.513 g/mol | 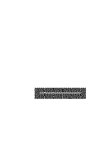 | 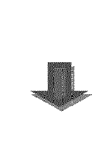 | 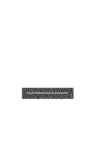 | 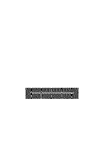 | 4 |
| | | | mass in vial: 140.1 mg | | | | | |
| Unknown Group 3 | 103 | 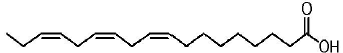 | linolenic acid MC 6-103 mw: 278.429 g/mol | 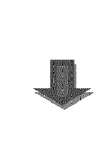 | 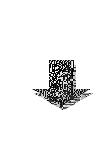 | 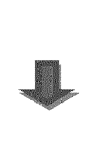 | 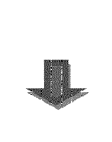 | 2,5 |
| | | | mass in vial: 110.6 mg | | | | | |
| | 57 | 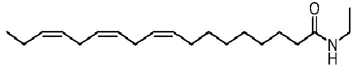 | N-ethyllinolenamide MC 6-57 mw: 305.498 g/mol | 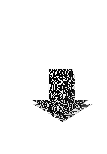 | 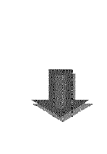 | 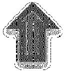 | 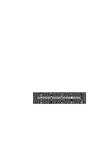 | 4 |
| | | | mass in vial: 26.7 mg | | | | | |
| Unknown Group 3 | 59 | 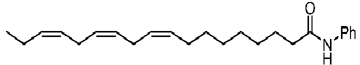 | N-phenyllinolenamide MC 6-59 mw: 353.540 g/mol | 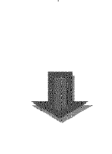 | 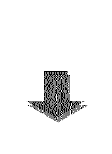 | 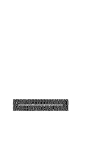 | 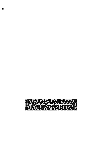 | 4 |
| | | | mass in vial: 39.9 mg | | | | | |
| | 60 | 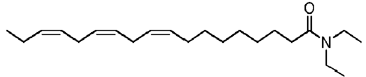 | N,N-diethyllinolenamide MC 6-60 mw: 333.551 g/mol | 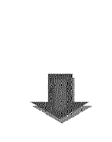 | 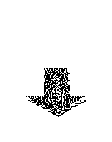 | 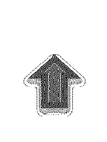 | 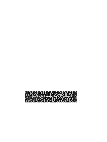 | 4 |
| | | | mass in vial: 25.2 mg | | | | | |
| | 62 | 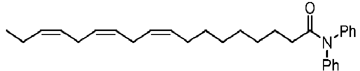 | N-ethyllinolenamide MC 6-62 mw: 429.636 g/mol | 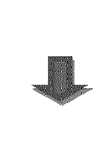 | 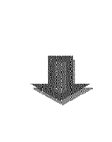 | 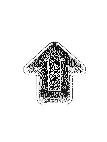 | 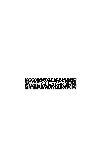 | 4 |
| | | | mass in vial: 25.4 mg | | | | | |
| | 99 | 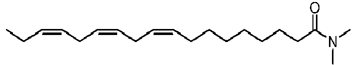 | N,N-dimethyllinolenamide MC 6-99 mw: 305.498 g/mol | 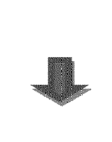 | 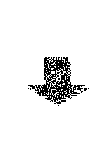 | 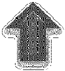 | 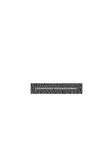 | 4 |
| | | | mass in vial: 44.2 mg | | | | | |

| | | | | | | | | |
|---|---|---|---|---|---|---|---|---|
| * There are five potential categories based on the modulation of the cytokines for each analogue. They are as follows: 1 - A parent analogue of the Oleamide compound where there is a decrease in IL-12p40, IL-12p70 and IL-23 But NOT in TNF-alpha 2 - A decrease in IL-12p40, IL-12p70, IL-23 and TNF-alpha 3 - Differential effects on IL-12p40, IL-12p70 and IL-23 and a Decrease in TNF-alpha 4 - Differential effects on IL-12p40, IL-12p70, IL-23 and TNF-alpha 5 - Anti-inflammatory activity | | | | | | | | |

The invention is not limited to the embodiments hereinbefore described but may be varied in both construction and detail.

## Claims

1. Oleamide, or an immunomodulatory analogue thereof, for use in a method of treating a peripheral inflammatory disorder in a mammal.

2. Oleamide, or an immunomodulatory analogue thereof, for use of Claim 1, in which the peripheral inflammatory disease is an immune-mediated inflammatory disorder of the skin.

3. Oleamide, or an immunomodulatory analogue thereof, for use of Claim 1, in which the immune-mediated inflammatory disorder of the skin is selected from atopic dermatitis, contact dermatitis, acne and psoriasis.

4. Oleamide, or an immunomodulatory analogue thereof, for use of any of Claims 1 to 3, in which the oleamide or immunomodulatory analogue thereof is administered topically to the skin.

5. Oleamide, or an immunomodulatory analogue thereof, for use of Claim 1, in which the peripheral inflammatory disease is an immune-mediated inflammatory disorder of the joints.

6. Oleamide, or an immunomodulatory analogue thereof, for use of Claim 5, in which the immune-mediated inflammatory disorder of the joints is rheumatoid arthritis.

7. Oleamide, or an immunomodulatory analogue thereof, for use of Claim 1, in which the peripheral inflammatory disease is an immune-mediated inflammatory disorder of the cardiovascular system.

8. Oleamide, or an immunomodulatory analogue thereof, for use of Claim 7, in which the immune-mediated inflammatory disorder is atherosclerosis.

9. Oleamide, or an immunomodulatory analogue thereof, for use of Claim 1, in which the peripheral inflammatory disease is an immune-mediated respiratory inflammatory disorder.

10. Oleamide, or an immunomodulatory analogue thereof, for use of Claim 9, in which the immune-mediated respiratory inflammatory disorder is asthma.

11. Oleamide, or an immunomodulatory analogue thereof, for use of Claim 1, in which the peripheral inflammatory disease is an immune-mediated inflammatory disorder of the intestinal tract.

12. Oleamide, or an immunomodulatory analogue thereof, for use of Claim 11, in which the immune-mediated inflammatory disorder of the intestinal tract is inflammatory bowel disease.

13. A pharmaceutical composition comprising oleamide or an immunomodulatory analogue thereof and a suitable pharmaceutical excipient, formulated for topical delivery to the skin of a human.

14. A pharmaceutical composition as claimed in Claim 14 formulated as a cream, ointment, paste, lotion, spray or gel.

15. A pharmaceutical composition as claimed in Claim 13 or 14 for use in a method of treating atopic dermatitis in a human.
